# EUROPEAN PATENT SPECIFICATION

(11) **EP 0 811 625 B1**
(45) Date of publication and mention of the grant of the patent: **17.04.2002**
(21) Application number: 97810324.0
(22) Date of filing: 27.05.1997
(51) Int. Cl.: C07D 487/04, C07D 519/00, C09B 57/00

(54) **Process for preparing diketopyrrolopyrrole derivatives**
Verfahren zur Herstellung von Diketopyrrolopyrrolderivaten
Procédé de préparation de dérivés diketopyrrolopyrroles

(30) Priority: 05.06.1996 US 19138 P; 26.09.1996 US 27469 P; 26.09.1996 US 27470 P
(43) Date of publication of application: 10.12.1997
(73) Proprietor: Ciba Specialty Chemicals Holding Inc., 4057 Basel (CH)
(72) Inventor: Hendi, Shivakumar Basalingappa, Newark, DE 19702 (US)

(56) References cited:
- EP-A- 0 133 156
- EP-A- 0 224 445
- EP-A- 0 648 770

## Description

This application relates to a process for preparing 1,4-diketo-3,6-diarylpyrrolopyrrole derivatives wherein a diketopyrrolopyrrole is reacted with formaldehyde or paraformaldehyde to yield a 2,5-bis(hydroxymethyl)-3,6-diarylpyrrolopyrrole-1,4-dione which is isolated or further reacted.

Further, this application relates to a process wherein a 2,5-bis(hydroxymethyl)-3,6-diarylpyrrolopyrrole-1,4-dione is reacted with a precursor of organic radicals B₁ and B₂, a process wherein the precursor of the organic radicals B₁ and B₂ is a 1,4-diketo-3,6-diarylpyrrolo[3,4-c]pyrrole or a quinacridone, a 2,5-bis(hydroxymethyl)-3,6-diarylpyrrolopyrrole-1,4-dione, a process for its preparation, a diketopyrrolopyrrole derivative having two quinacridone radicals as substituents, a diketopyrrolopyrrole derivative having two diaryldiketopyrrolopyrrole radicals as substituents, a pigment composition, a method of reducing the viscosity of a dispersion, and a method of preparing a basecoat/clearcoat finish.

Diaryldiketopyrrolopyrroles of the formula 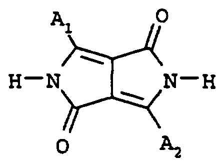 wherein A₁ and A₂ are aryl radicals are well-known as important pigments.

U.S. Patent No. 4,585,878 discloses N-substituted derivatives of the diaryldiketopyrrolopyrrole pigments, which are useful as polymer soluble dyes or as pigments, wherein the N-substituent does not confer solubility in water. According to U.S. Patent No. 4,585,878, the N-substituted derivatives of the diaryldiketopyrrolopyrrole pigments are prepared by reacting a diaryldiketopyrrolopyrrole compound in an organic solvent with a compound containing the ultimate N-substituents attached to a leaving group, or by reacting 2 moles of a compound of the formula R-N=CH-A, wherein R is the N-substituent and A is an aryl group, or one mole each of two different compounds of the formula R-N=CH-A, with a succinic acid diester in the presence of a base and an organic solvent and then dehydrogenating the product.

Rheology-improving agents for organic pigments reduce the viscosity of a dispersion of the organic pigment in a high-molecular-weight organic material. Some compounds capable of functioning as rheology-improving agents for organic pigments are known. For example, phthalimidomethylquinacridone, quinacridone monosulfonic acid salts, especially the aluminum salt, the dimethylaminopropylsulfonamide derivative of quinacridone and pyrrazolylmethylquinacridone are known rheology improving agents for organic pigments.

Although the known rheology improving agents adequately reduce the viscosity when the organic pigment is dispersed in a high-molecular-weight organic material, the incorporation of the known rheology improving agents in a pigment composition based on a bright red or highly saturated orange pigment generally results in unacceptable pigmentary properties with significant loss in color and color saturation.

Hence, it was the object of the present invention to provide rheology improving agents which do not show the abovementioned drawbacks. Preferably, the rheology improving agents should be based on N-substituted diaryldiketopyrrolopyrroles.

Accordingly a process for the preparation of a diketopyrrolopyrrole derivative of the formula (I) 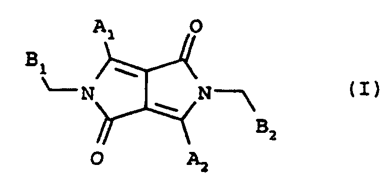 was found, wherein A₁ and A₂ are radicals of the formula 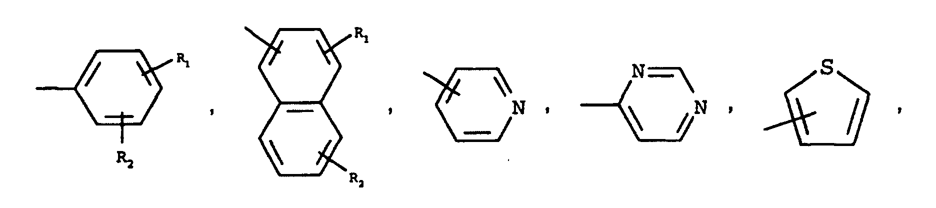 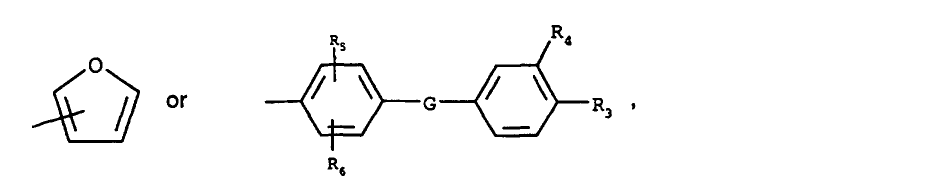 wherein
R₁ and R₂ are each independently of the other hydrogen, halogen, C₁-C₁₈alkyl, C₁-C₁₈alkoxy, C₁-C₁₈alkylmercapto, C₁-C₁₈alkylamino, C₁-C₁₈alkoxycarbonyl, C₁-C₁₈alkylaminocarbonyl, -CN, -NO₂, trifluoromethyl, C₅-C₆cycloalkyl, -C=N-(C₁-C₁₈alkyl), phenyl, 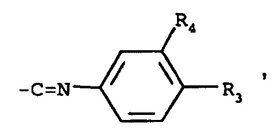 midazolyl, pyrrazolyl, triazolyl, piperazinyl, pyrrolyl, oxazolyl, benzoxazolyl, benzothiazolyl, benzimidazolyl, morpholinyl, piperidinyl or pyrrolidinyl,
G is -CH₂-, -CH(CH₃)-, -C(CH₃)₂-, -CH=N-, -N=N-, -O-, -S-, -SO-, -SO₂-, -CONH- or - NR₇-,
R₃ and R₄ are each independently of the other hydrogen, halogen, C₁-C₆alkyl, C₁-C₁₈alkoxy or -CN, R₅ and R₆ are each independently of the other hydrogen, halogen or C₁-C₆alkyl, and R₇ is hydrogen or C₁-C₆alkyl, and B₁ and B₂ are identical or different organic radicals which derive from a precursor which reacts with the hydroxy groups of a compound of formula (II) either by substitution reaction or to form an -O-linkage; wherein the precursor which reacts with a hydroxyl group by a substitution reaction is a compound which is selected from the group consisting of an aromatic radical, a heteroaromatic radical, or radicals of the formulae X-C(=O)-Y-, X-C(=O)-Y-, -Y-C(=O)-O-X, X-C(=S)-Y-, - Y-C(=S)-O-X, -Y-C(=S)-S-X, X-C(=N)-Y-, XZN-, X-S-, X-SO₂-, X-SO₂-NR₁₀-, 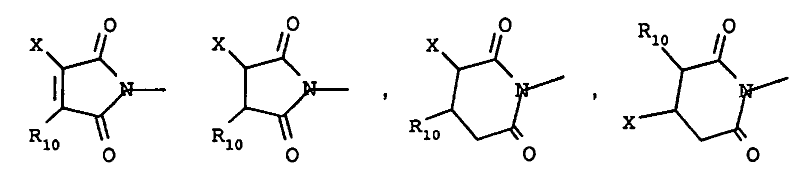 and -N(R₁₀)-C(=O)-O-X; wherein X and Z are each hydrogen or an aliphatic, alicyclic, araliphatic, aromatic or heterocyclic radical, or X and Z together form a 3 to 8 membered ring, Y is the residue of an active methylene containing moiety, and R₁₀ is hydrogen, an aliphatic radical, an alicyclic radical, an araliphatic radical, an aromatic radical or a heterocyclic radical, or R₁₀ and X together form a 3 to 8 membered ring, and the precursor which reacts with a hydroxyl group to form an -O- linkage is selected form the group consisting of an alkyl halide and radicals of the formulae X-C(=O)O-, X-C(=S)O-, X-SO₂-O- and XO-; wherein X has the meaning given above,
which diketopyrrolopyrrole derivative contains from 0 to 6 moles of -SO₃M per mole of diketopyrrolopyrrole derivative; wherein M is hydrogen or a metal or ammonium cation, which process comprises a reaction wherein a 1,4-diketo-3,6-diarylpyrrolopyrrole of the formula 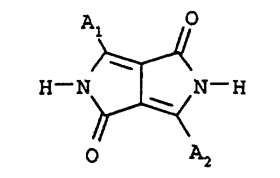 is reacted in a first step with formaldehyde or paraformaldehyde to yield a sulfonated or non-sulfonated intermediate of the formula 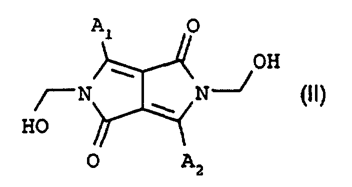 which reacts in a second step with a precursor of the organic radicals, B₁ and B₂, to yield the diketopyrrolopyrrole derivative of formula (I).

Further, a process, wherein a 2,5-bis(hydroxymethyl)-3,6-diarylpyrrolopyrrole-1,4-dione is reacted with a precursor of organic radicals B₁ and B₂, a process wherein the precursor of the organic radicals B₁ and B₂ is a 1,4-diketo-3,6-diarylpyrrolo[3,4-c]pyrrole or a quinacridone, a 2,5-bis(hydroxymethyl)-3,6-diarylpyrrolopyrrole-1,4-dione, a process for its preparation, a diketopyrrolopyrrole derivative having two quinacridone radicals as substituents, a diketopyrrolopyrrole derivative having two diaryldiketopyrrolopyrrole radicals as substituents, a pigment composition, a method of reducing the viscosity of a dispersion, and a method of preparing a basecoat/clearcoat finish were found, too.

The present invention relates to a process for preparing a variety of N-substituted derivatives of diaryldiketopyrrolopyrrole pigments wherein the N-substituents are linked to the diaryldiketopyrrolopyrrole by -CH₂- or -O-CH₂- linkages. The inventive process involves reacting a diaryldiketopyrrolopyrrole with formaldehyde or paraformaldehyde to yield a 2,5-bis(hydroxymethyl)-3,6-diarylpyrrolopyrrole-1,4-dione intermediate and further reacting the intermediate, with or without isolation, with a second reactant which reacts with the hydroxymethyl groups. The products of the reaction are useful as colorants and as rheology improving agents for pigment dispersions. The 2,5-bis(hydroxymethyl)-3,6-diarylpyrrolopyrrole-1,4-dione intermediate is useful as a synthon for the preparation of a variety of DPP derivatives, and as a stabilizer for polymers.

In general, the second reactant reacts with the compound of formula (II) by a substitution reaction or to form an -O- linkage.

The first step is preferably carried out by adding the 1,4-diketo-3,6-diarylpyrrolo[3,4-c]pyrrole to a solution of paraformaldehyde in concentrated sulfuric acid, preferably having a H₂SO₄ concentration greater than 90 percent by weight, most preferably above 95 percent by weight.

In general, the stoichiometric amount of formaldehyde or paraformaldehyde (calculated as formaldehyde) is used in the first step. Thus, the molar ratio of the 1,4-diketo-3,6-diarylpyrrolo[3,4-c]pyrrole to the formaldehyde or paraformaldehyde (calculated as formaldehyde) during the first step usually is in the range of 0.75:2 to 1.25 to 2, preferably 1:2.

After the first step is complete, the resulting intermediate is reacted with the precursor to yield the diketopyrrolopyrrole derivative of formula (I).

Preferably, both steps are carried out at a temperature of from 20 to 100°C. If a high degree of sulfonation is desired, the process is carried out at higher temperatures, for example above 40°C. If it is desirable to have a low degree of sulfonation, the reaction is maintained at a lower temperature, preferably 40°C or below.

After the reaction is complete, the 1,4-diketo-3,6-diarylpyrrolo[3,4-c]pyrrole derivative usually is isolated by procedures conventionally used in the art for isolating 1,4-diketo-3,6-diarylpyrrolo[3,4-c]pyrroles, in particular by pouring the sulfuric acid solution into ice water maintaining the temperature below 10°C and stirring the resulting aqueous slurry for about 1 hour, followed by filtration, washing and drying to yield the 1,4-diketo-3,6-diarylpyrrolo[3,4-c]pyrrole derivative in solid form.

Since it is not necessary to isolate the intermediate, the inventive process is preferably a one pot process. However, it is possible to isolate the intermediate prior to carrying out the second step, especially, for example, in those instances where it is desired to carry out the second step in a solvent other than the solvent used for the first step.

Suitable solvents for the second step include concentrated sulfuric acid, polyphosphoric acid and organic solvents which do not react with the compound of formula (II), especially polar organic solvents such as acetonitrile, benzonitrile, dimethylformamide, dimethylsulfoxide, tetramethylenesulfone.

It is also possible to carry out the second step in a solvent which reacts with the compound of formula (II) to yield the desired product, for example, C₁-C₁₀ alcohols are suitable solvents if the compound of formula (I) is the ether obtainable by reacting the compound of formula (II) with the alcohol.

In a preferred embodiment of the present invention a 1,4-dlketo-3,6-diarylpyrrolopyrrole of the formula (II) is reacted with a precursor of the organic radicals, B₁ and B₂.

Preferably, the diketopyrrolopyrrole derivatives of formula (I) contain from 0 to 4 moles of -SO₃M per mole of diketopyrrolopyrrole derivative; most preferably from 0 to 2 moles per mole of diketopyrrolopyrrole derivative. In general, if the reaction is carried out at about 40-50°C, the product contains about 0.5 moles of -SO₃M per mole of diketopyrrolopyrrole derivative.

M is preferably hydrogen, or an alkali metal, such as sodium or potassium, an alkaline earth metal, such as magnesium, an aluminum, a zinc or an ammonium cation. Examples of suitable ammonium cations include quaternary ammonium cations, such as trimethylcetylammonium or tributylbenzylammonium.

A₁ and A₂ include radicals of the formula 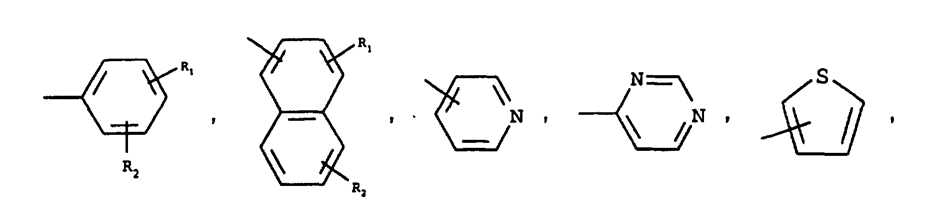 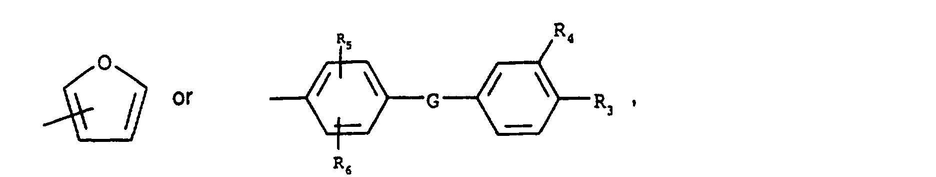 wherein
R₁ and R₂ are each independently of the other hydrogen, halogen, C₁-C₁₈alkyl, C₁-C₁₈alkoxy, C₁-C₁₈alkylmercapto, C₁-C₁₈alkylamino, C₁-C₁₈alkoxycarbonyl, C₁-C₁₈alkylaminocarbonyl, -CN, -NO₂, trifluoromethyl, C₅-C₆cycloalkyl, -CH=N-(C₁-C₁₈alkyl), phenyl, 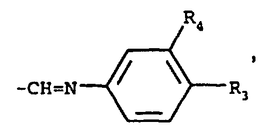 imidazolyl, pyrrazolyl, triazolyl, piperazinyl, pyrrolyl, oxazolyl, benzoxazolyl, benzothiazolyl, benzimidazolyl, morpholinyl, piperidinyl or pyrrolidinyl,
G is -CH₂-, -CH(CH₃)-, -C(CH₃)₂-, -CH=N-, -N=N-, -O-, -S-, -SO-, -SO₂-, -CONH- or -NR₇-, R₃ and R₄ are each independently of the other hydrogen, halogen, C₁-C₆alkyl, C₁-C₁₈alkoxy or -CN, R₅ and R₆ are each independently of the other hydrogen, halogen or C₁-C₆alkyl, and R₇ is hydrogen or C₁-C₆alkyl.

In particular, A₁ and A₂ are each a group of formula 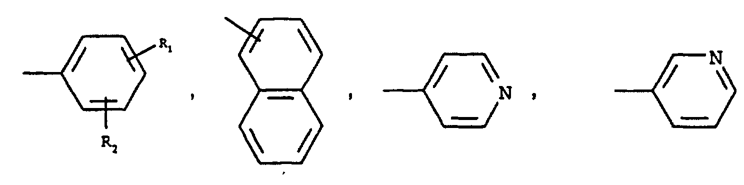 or 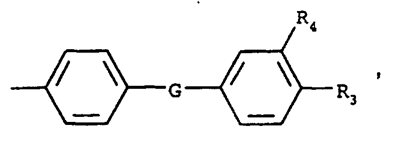 wherein
R₁ and R₂ are each independently of the other hydrogen, chloro, bromo, C₁-C₄alkyl, C₁-C₆alkoxy, C₁-C₆alkylamino, phenyl or CN,
G is -O-, -NR₇-, -N=N- or -SO₂-,
R₃ and R₄ are hydrogen, and R₇ is hydrogen, methyl or ethyl,
and more particularly A₁ and A₂ are each a group of formula 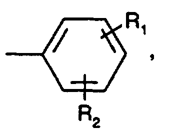 wherein R₁ and R₂ are each independently of the other hydrogen, methyl, tert-butyl, chloro, bromo, phenyl or CN. At least one of R₁ and R₂ is preferably hydrogen. Most preferably, at least one of R₁ and R₂ is hydrogen and the other is in the 3- or 4 position of the phenyl ring.

According to the present invention, the organic radicals B₁ and B₂ are derivable from a precursor which reacts with the hydroxy groups of a compound of the formula (II) either by a substitution reaction or to form an -O-linkage.

Precursors which react with a hydroxyl group by a substitution reaction are generally compounds which comprise an aromatic radical, a heteroaromatic radical, or radicals of the formulae X-C(=O)-Y-, -Y-C(=O)-O-X, X-C(=S)-Y-, -Y-C(=S)-O-X, -Y-C(=S)-S-X, X-C(=N)-Y-, XZN-, X-S-, X-SO₂-, X-SO₂-NR₁₀-, 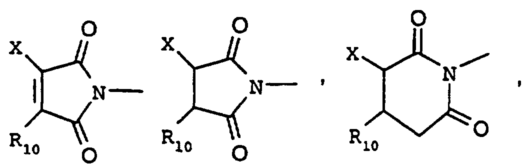 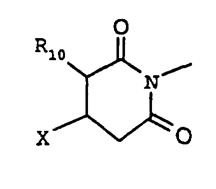 and -N(R₁₀)-C(=O)-O-X; wherein X and Z are each hydrogen or an aliphatic, alicyclic, araliphatic, aromatic or heterocyclic radical, or X and Z together form a 3 to 8 membered ring, Y is the residue of an active methylene containing moiety, and R₁₀ is hydrogen, an aliphatic radical, an alicyclic radical, an araliphatic radical, an aromatic radical or a heterocyclic radical, or R₁₀ and X together form a 3 to 8 membered ring.

Examples of B₁ and B₂ radicals derivable from a precursor which reacts with a hydroxyl group to form an -O-linkage include an alkyl halide and radicals of the formulae X-C(=O)O-, X-C(=S)O-, X-SO₂-O-, XO-; wherein X has the meaning given above.

As aromatic radicals, B₁ and B₂ especially include radicals containing 1, 2, 3, 4 or more phenyl rings which are bonded directly to each other, bonded to each other through a linking group, fused, or any combination thereof. The biphenylyl radical is an example of two phenyl rings directly bonded to each other. Radicals of the formula 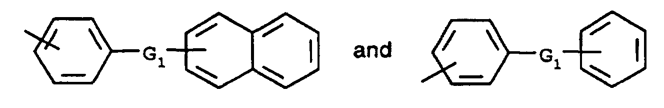 wherein G₁ is a linking group, are examples of phenyl rings bonded through each other through a linking group. Examples of aromatic radicals containing fused phenyl rings include naphthyl, anthryl and phenanthryl.

The linking group G₁ is especially those linking groups described above for the variable G.

As heteroaromatic radicals, B₁ and B₂ especially include radicals containing one or more 5, 6 or 7 membered aromatic rings containing from 1 to 4 heteroatoms. In general, the heteroatom(s) are nitrogen, oxygen, sulfur or any combination thereof. Suitable heteroaromatic radicals include the pyrrolopyrroles, especially the 1,4-diketo-3,6-diarylpyrrolo[3,4-c]pyrroles and quinacridones.

Preferably, Y is the residue of an active methylene containing moiety. Active methylene moieties are generally those methylene groups which are linked to an electron withdrawing substituent, such as a carbonyl or nitrite substitutent. In general, active methylene moieties participate in a Mannich type or similar reaction. In general, Y is -CHX, -CH₂-X, -(CH=CH)ₙ-CH₂-X, wherein n is 1, 2 or 3. When Y is the -CHX- radical, the -CHX- radical is part of a ring, for example, when B₁ or B₂ is the X-C(=O)-Y- radical, X and Y can form part of for example, a cyclohexanone ring.

X and Z usually are aliphatic, alicyclic, araliphatic, aromatic or heterocyclic radicals.

In general, aliphatic radicals include C₁-C₁₀ alkyl, C₁-C₁₀ alkenyl, and C₁-C₁₀ alkynyl radicals, including straight and branched chains.

Alicyclic radicals include those moieties containing only one or more nonaromatic hydrocarbon rings. Important alicyclic radicals include radicals derived from C₃-C₈ cycloalkanes and C₃-C₈cycloalkenes. Examples of important alicyclic radicals include cyclopentyl, cyclohexyl and cycloheptyl. Alicyclic radicals also include those moieties wherein there is, for example, a -C(=O)- on the ring, such as cyclohexanone.

Araliphatic radicals are those moieties which contain an aliphatic portion and an aromatic portion, for example a phenyl or heteroaromatic portion. Examples of araliphatic radicals include radicals derived from the phenylalkanoic acids, the naphthylalkanoic acids, the pyridine alkanoic acids, the quinoline alkanoic acids, the indole alkanoic acids, such as those derived from phenyl acetic acid, phenyl propionic acid, or indole acetic acid.

Aromatic radicals suitable as X and Z include those described above as being useful as B₁ and B₂. The term "aromatic" radical, in this instance, does not include heteroaromatic radicals.

Heterocyclic radicals contain one or more nonaromatic and/or aromatic rings which contain one or more heteroatoms; especially 3 to 8 membered rings which contain 1 to 3 heteroatoms, which heteroatoms are especially nitrogen, sulfur and oxygen. The term heterocyclic radical includes fused ring systems wherein one or more rings contain one or more heteroatoms. Important heterocyclic radicals include pyridinyl, pyranyl, tetrahydrofuranyl, morpholino, pyrimidyl, pyrone, oxazine, azepinyl, triazinyl, oxathiazinyl, pyrroyl, benzofuranyl, piperazinyl, oxathiazolyl, oxadiazolyl, quinolinyl, indolyl, carbazolyl, xanthenyl, acridinyl, coumarinyl, benzoxazolyl, benzopyrone, quinazolinyl. Heterocyclic radicals include those wherein the ring is a lactone or a lactam.

The description of radicals above also defines aliphatic, alicyclic, araliphatic, aromatic and heterocyclic radicals suitable as R₁₀. When R₁₀ combines with X to form a ring, the ring is preferably a five or six membered ring.

The aromatic, heterocyclic, aliphatic, alicyclic and araliphatic radicals are unsubstituted (by any group other than hydrogen) or substituted by one or more, preferably 0 to 4, customary substituents.
Customary substituents include hydroxy, carbonyl, halogen, C₁-C₁₈alkyl, C₁-C₁₈alkoxy, C₁-C₁₈alkylmercapto, C₁-C₁₈alkylamino, di(C₁-C₁₈alkyl)amino, C₁-C₁₈alkoxycarbonyl, C₁-C₁₈alkylaminocarbonyl, -CN, -NO₂, trifluoromethyl, C₅-C₆cycloalkyl, -C=N-(C₁-C₁₈alkyl), phenyl, wherein alkyl groups can be further substituted by hydroxyl, halogen, nitro, C₁-C₆alkoxy, carbonyl, -CN.

Substituents defined as halogen are typically iodo, fluoro, bromo and, preferably, chloro;

C₁-C₆alkyl is typically methyl, ethyl, n-propyl, isopropyl, n-butyl, sec-butyl, tert-butyl, n-amyl, tert.-amyl, hexyl, and C₁-C₁₀alkyl and C₁-C₁₈alkyl are in addition typically heptyl, octyl, 2-ethylhexyl, nonyl, decyl, dodecyl, tetradecyl, hexadecyl or octadecyl.

C₁-C₁₈Alkoxy, also in C₁-C₁₈alkoxycarbonyl, is typically methoxy, ethoxy, n-propoxy, isopropoxy, butoxy, hexyloxy, decyloxy, dodecyloxy, hexadecyloxy or octadecyloxy.

C₁-C₁₈Alkylmercapto is, for example, methylmercapto, ethylmercapto, propylmercapto, butylmercapto, octylmercapto, decylmercapto, hexadecylmercapto or octadecylmercapto.

C₁-C₁₈Alkylamino is, also in C₁-C₁₈alkylaminocarbonyl, typically methylamino, ethylamino, propylamino, hexylamino, decylamino, hexadecylamino or octadecylamino.

C₅-C₆Cycloalkyl is typically cyclopentyl and cyclohexyl.

Important radicals containing the residue of an active methylene, Y, include radicals derivable from various acetoacetanilides, cyanoacetanilides and benzoylacetanilides, such as ethylacetoacetate, ethyl malonate, ethyl cyanoacetate, ethyl benzoyl acetate and malononitrile.

Important radicals of the formula X-C(=O)-O- include esters derivable from C₁-C₂₄ aliphatic acids, such as acetic acid, stearic acid, oleic acid, linoleic acid, acrylic acid, methacrylic acid or trifluoroacetic acid, a C₁-C₂₄araliphatic acid, such as benzoic acid, phenyl acetic acid, phenyl propionic acid or indole acetic acid, a resin acid, such as abeitic acid, behemic acid, a naphthenic acid, a dimeric acid, wherein the aliphatic acids and araliphatic acids are unsubstituted or substituted by one or more customary substituents.

Important radicals of the formula X-C(=O)NR₁₀- and X-SO₂-NR₁₀- include those derivable from amides and sulfonamides prepared from an aliphatic amine and an aliphatic carboxylic or sulfonic acid, an aromatic amine and a aliphatic carboxylic or sulfonic acid, or an aromatic amine and an aromatic or araliphatic carboxylic or sulfonic acid. Suitable radicals of the formula X-C(=O)NR₁₀- and X-SO₂-NR₁₀ especially include those wherein X is C₁-C₂₄ alkyl, phenyl, benzyl, tolyl, naphthyl and R₁₀ is hydrogen, methyl, ethyl, n-propyl, isopropyl, phenyl, benzyl.

Important radicals of the formulae X-SO₂-, X-SO₂-O- and X-C(=S)-O- include those wherein X is a C₁-C₂₄ aliphatic radical, an C₆-C₁₈ aromatic radical, a C₁-C₂₄ araliphatic radical, a 5, 6 or 7 membered heterocyclic ring, or a fused ring system containing a 5, 6 or 7 membered heterocyclic ring, such as pentyl, hexyl, phenyl, benzyl, tolyl, naphthyl, pyridinyl or indolyl. Important radicals of the formula X-SO₂-O- include those derivable from p-toluene sulfonic acid, naphthalene sulfonic acid, pentane sulfonic acid or a water-soluble dye which contains a -SO₃H water-solublizing group.

Important radicals of the formula XZN- include those derivable from N,N-di C₁-C₂₄alkyl amines, such as dimethylamine, diethylamine dipropylamine, and dibutylamine, arylamines, such as N,N-diphenylamine, aralkylamines, such as N,N-dibenzylamine or ethylphenylamine, or heteroaryl amines, such as aminopyridine. The formula XZN- also includes those radicals wherein X and Z, together, along with the nitrogen atom, form a 3 to 8 membered ring, especially a 5 or 6 membered ring, such as a piperazine, morpholine, thiomorpholine, pyrrolidine or piperidine ring.

Important radicals of the formulae XO- include those wherein X is a C₁-C₂₄ aliphatic radical, a C₅-C₁₀ alicyclic radical, phenyl, benzyl, naphthyl, or a radical H-(CH₂CHR₁₁O)ₘ-CH₂CHR₁₁-, wherein R₁₁ is hydrogen or methyl, especially hydrogen, and m is a number from 1 to 20.

Important radicals of the formula XS- include those wherein X is a C₁-C₂₄ aliphatic radical, a C₅-C₁₀alicyclic radical, phenyl, benzyl and naphthyl.

Important radicals of the formula 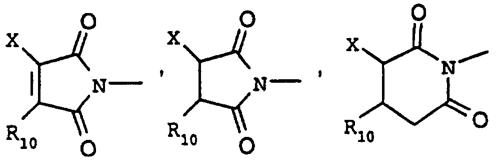 or 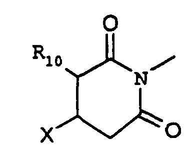 include radicals derivable from succinimide, glutarimide, phthalimide, naphthalimide and isoquinoline-1,3-dione.

Important radicals of the formula -N(R₁₀)-C(=O)-O-X include those wherein R₁₀ is hydrogen, C₁-C₆alkyl, phenyl or benzyl, especially hydrogen and C₁-C₆alkyl, and X is C₁-C₆alkyl, for example, urethane.

Aromatic and heteroaromatic radicals derivable from known dyes, such as azo, azomethine, or fiber-reactive dyes, for example triazine dyes, or known organic pigments, such as diketopyrrolopyrrole, quinacridone, phthalocyanine, indanthrone, isoindoline, isoindolone, flavanthrone, pyranthrone, anthraquinone, thioindigo, perylene and dioxazine pigments are suitable radicals for B₁ and B₂. Quinacridinyl (derived from a quinacridone) and 1,4-diketo-3,6-diarylpyrrolo[3,4-c]pyrrolyl (derived from a 1,4-diketo-3,6-diarylpyrrolo[3,4-c]pyrrole) radicals are especially suitable radicals for B₁ and B₂.

Thus, an aspect of the present invention relates to a process of preparing diketopyrrolopyrrole derivatives of the formula (I) wherein B₁ and B₂ are each a pigment moiety, in particular, a diketopyrrolopyrrole, quinacridone, phthalocyanine, indanthrone, isoindoline, isoindolone, flavanthrone, pyranthrone, anthraquinone, thioindigo, perylene and dioxazine pigment moiety.

1,4-diketo-3,6-diarylpyrrolo[3,4-c]pyrrolyl radicals are especially suitable radicals for B₁ and B₂. Such radicals are derivable from compounds of the formula 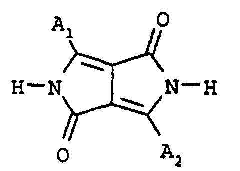 which are well-known as pigments, preferably 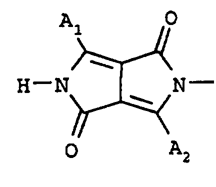 is used as radical.

When B₁ and B₂ are 1,4-diketo-3,6-diarylpyrrolo[3,4-c]pyrrolyl radicals, the preferences described above for A₁ and A₂ apply.

Another aspect of this invention relates to a process for preparing diketopyrrolopyrrole derivatives of formula (I) wherein B₁ and B₂ are quinacridinyl radicals. Such radicals are derivable from the compounds of the formula 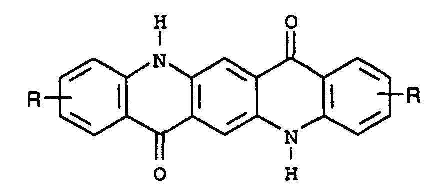 wherein each R is independently hydrogen, halogen, carboxyl, unsubstituted C₁-C₆ alkyl, C₁-C₆ alkyl which is substituted by halogen, unsubstituted C₁-C₆ alkoxy, or C₁-C₆ alkoxy which is substituted by halogen.

B₁ and B₂ are especially radicals derivable from quinacridone, 2,9-dichloroquinacridone, 4,11-dichloroqunacridone, 2,9-dimethylquinacridone, 4,11-dimethylquinacridone, 2,9-difluoroquinacridone.

The present invention also relates to a compound of the formula 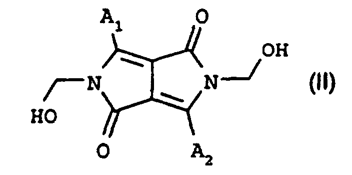 which contains from 0 to 6 moles of -SO₃M per mole of the compound, wherein A₁ and A₂ are as defined above and M is hydrogen or a metal or ammonium ration.

A₁ and A₂ have the meaning given above.

Preferably A₁ and A₂ are radicals of the formula 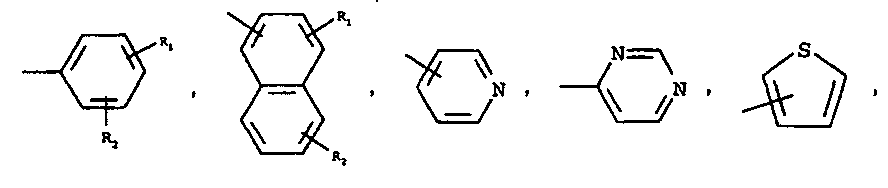 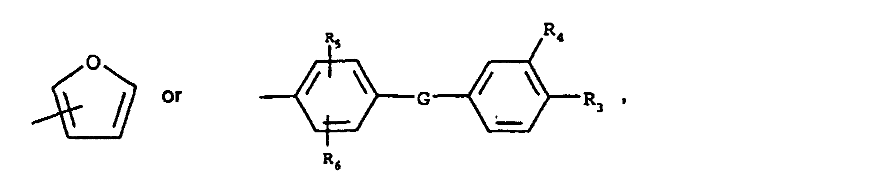 wherein
R₁ and R₂ are each independently of the other hydrogen, halogen, C₁-C₁₈alkyl, C₁-C₁₈alkoxy, C₁-C₁₈alkylmercapto, C₁-C₁₈alkylamino, C₁-C₁₈alkoxycarbonyl, C₁-C₁₈alkylaminocarbonyl, -CN, -NO₂, trifluoromethyl, C₅-C₆cycloalkyl, -CH=N-(C₁-C₁₈alkyl), phenyl, 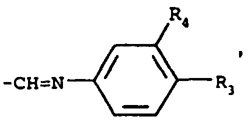 imidazolyl, pyrrazolyl, triazolyl, piperazinyl, pyrrolyl, oxazolyl, benzoxazolyl, benzothiazolyl, benzimidazolyl, morpholinyl, piperidinyl or pyrrolidinyl,
G is -CH₂-, -CH(CH₃)-, -C(CH₃)₂-, -CH=N-, -N=N-, -O-, -S-, -SO-, -SO₂-, -CONH- or -NR₇-,
R₃ and R₄ are each independently of the other hydrogen, halogen, C₁-C₆alkyl, C₁-C₁₈alkoxy or -CN, R₅ and R₆ are each independently of the other hydrogen, halogen or C₁-C₆alkyl, and R₇ is hydrogen or C₁-C₆alkyl.

In particular, A₁ and A₂ are radicals of the formula 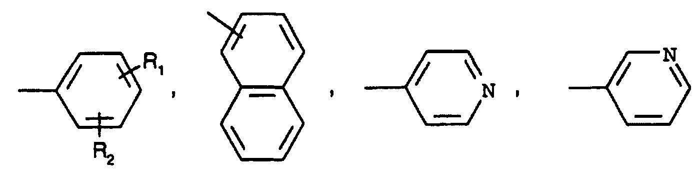 or 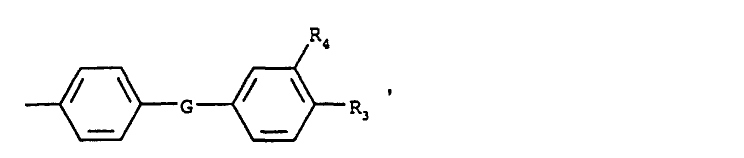 wherein
R₁ and R₂ are each independently of the other hydrogen, chloro, bromo, C₁-C₄alkyl, C₁-C₆alkoxy, C₁-C₆alkylamino, phenyl or CN,
G is -O-, -NR₇-, -N=N- or -SO₂-,
R₃ and R₄ are hydrogen, and
R₇ is hydrogen, methyl or ethyl.

Most preferably, A₁ and A₂ are radicals of the formula 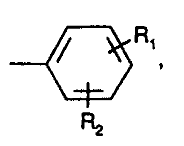 wherein R₁ and R₂ are each independently of the other hydrogen, methyl, tert-butyl, chloro, bromo, phenyl or CN. Preferably, at least one of R₁ and R₂ is hydrogen.

Preferably the compounds of formula (II) have from 0 to 4 moles of -SO₃M, most preferably from 0 to 2 moles of -SO₃M.

The present invention further relates to a process for preparing a compound of the formula 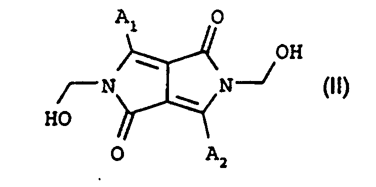 which contains from 0 to 6 moles of -SO₃M per mole of the compound, wherein A₁ and A₂ are as defined above and M is hydrogen or a metal or ammonium cation, which process comprises reacting a 1,4-diketo-3,6-diarylpyrrolopyrrole of the formula 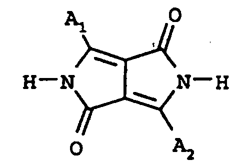 with formaldehyde or paraformaldehyde.

The preferences discussed above relating to the first step of the process for preparing the compounds of formula (I) are applicable to this aspect of the invention, too.

In particular, the present invention relates to the process wherein the 1,4-diketo-3,6-diarylpyrrolopyrrole is combined with paraformaldehyde in concentrated sulfuric acid; especially wherein the 1,4-diketo-3,6-diarylpyrrolopyrrole and formaldehyde (or paraformaldehyde, caculated as formaldehyde) are present in a molar ratio in the range of 0.75:2 to 1.25:2, most preferably of about 1:2.

If less sulfonation is desired, the reaction preferably is carried out at a temperature of 40°C or below. The reaction generally proceeds at higher temperatures, for example, in the range from about 20 to 120°C, but higher temperatures generally lead to a higher degree of sulfonation; which may be desirable depending on the intended use of the final product.

The hydroxymethyl derivative of formula (II) is generally isolated by precipitation and filtration, for example by drowning in an organic solvent or water followed by filtration.

Another preferred aspect of the present invention relates to a diketopyrrolopyrrole derivative of the formula (la) (B₁ and B₂ = QA) 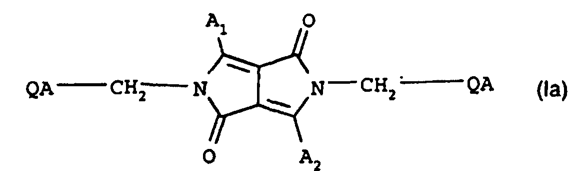 wherein A₁ and A₂ have the meaning given above, and QA is a quinacridone radical; which pyrrolopyrrole derivative is substituted by from 0 to 6 moles of -SO₃M per mole of the pyrrolopyrrole derivative; wherein M is hydrogen or a metal or ammonium cation as previously defined.

Preferably, the pyrrolopyrrole derivative is substituted with from 0 to 2 moles of -SO₃M per mole of DPP derivative, most preferably 0 to 0.75 moles of -SO₃M per mole of DPP derivative. Particularly preferred pyrrolopyrrole derivatives contain virtually no -SO₃M.

The preferences described above for A₁ and A₂ apply. In a preferred embodiment of the present invention A₁ and A₂ are phenyl or 4-tertiarybutylphenyl.

QA is preferably a quinacridone radical of the formula (III) 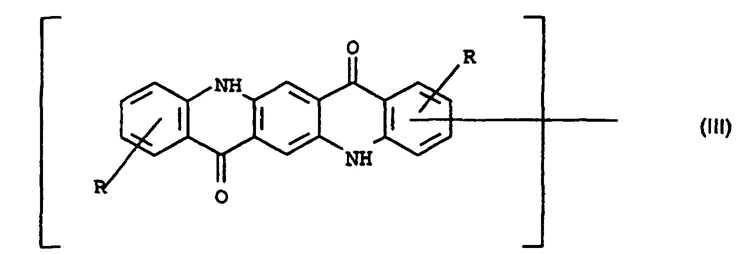 wherein each R is a customary substituent; preferably in the 2 and 9 or 4 and 11 positions.

Preferably, each R is independently hydrogen, halogen, carboxyl, unsubstituted C₁-C₆alkyl, C₁-C₆ alkyl which is substituted by halogen, unsubstituted C₁-C₆alkoxy, or C₁-C₆-alkoxy which is substituted by halogen.

Preferably, QA is a quinacridone radical derivable from quinacridone, 2,9-dichloroquinacridone, 4,11-dichloroquinacridone, 2,9-dimethylquinacridone, 4,11-dimethylquinacridone, 2,9-difluoroquinacridone. Thus, preferred QA substituents are quinacridone radicals of formula (III) wherein each R is hydrogen, Cl, F or -CH₃. Preferably, if both R substituents are not hydrogen, both R substituents are the same and selected from the group consisting of Cl, F or -CH₃ and most preferably in the 2, 9 or 4, 11 positions. Alternatively, both R substituents are hydrogen.

Especially important compounds are those wherein A₁ and A₂ are identical and are selected from the group consisting of phenyl, 4-methylphenyl, 4-tert-butylphenyl, 4-chlorophenyl, 4-bromophenyl and biphenyl-1-yl (4-phenyl-phenyl); especially those compounds wherein both R substituents on the QA radical of formula (III) are hydrogen.

In a particularly preferred embodiment of the present invention, the most preferred compounds are those pyrrolopyrrole derivatives of formula (la) wherein A₁ and A₂ are each a group of formula 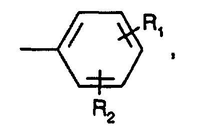 wherein R₁ and R₂ are each independently of the other hydrogen, methyl, tert-butyl, chloro, bromo, phenyl or CN;
and QA is a radical of the formula 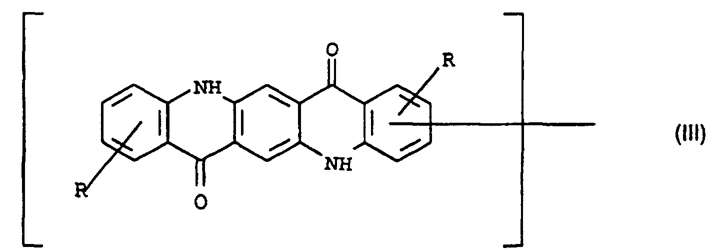 wherein the R substituents are hydrogen, 2,9- or 4,11-dichloro, 2,9- or 4,11-difluoro or 2,9- or 4,11-dimethyl.

Another preferred aspect of the present invention relates to a pyrrolopyrrole derivative of the formula (Ib) (B₁ and B₂ = DPP) 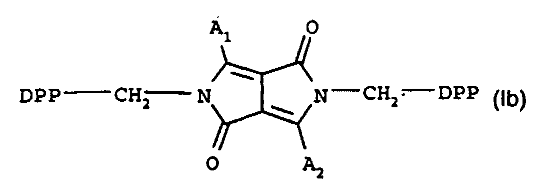 wherein A₁ and A₂ have the meaning given above, and DPP is a diaryldiketopyrrolopyrrole radical; which diketopyrrolopyrrole derivative is substituted by from 0 to 6 moles of -SO₃M per mole of the pyrrolopyrrole derivative; wherein M is hydrogen or a metal or ammonium cation.

Preferably, the pyrrolopyrrole derivative is substituted with from 0 to 2 moles of -SO₃M per mole of DPP derivative, most preferably 0 to 0.75 moles of -SO₃M per mole of DPP derivative. Particularly preferred pyrrolopyrrole derivatives contain virtually no -SO₃M.

Preferably, DPP is a diaryldiketopyrrolopyrrole radical of the formula 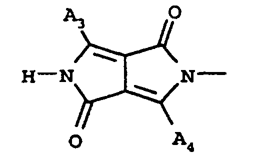 wherein A₃ and A₄ have the same meaning as A₁ and A₂ which are defined above.

The preferences described above for A₁ and A₂ apply, too, for A₃ and A₄.

Preferred compounds are those wherein A₁ and A₂ are identical and are selected from the group consisting of phenyl, 4-methylphenyl, 4-tert-butylphenyl, 4-chlorophenyl, 4-bromophenyl and biphenyl-1-yl (4-phenyl-phenyl), especially those wherein A₃ and A₄ are also identical and selected from the group consisting of phenyl, 4-methylphenyl, 4-tert-butylphenyl, 4-chlorophenyl, 4-bromophenyl and biphenyl-1-yl.

A preferred class of pyrrolopyrrole derivatives are those wherein A₃ and A₄ are each 4-tertbutylphenyl; especially those wherein A₁ and A₂ are identical and selected from phenyl, 4-tertbutylphenyl and 4-methylphenyl; especially 4-tertbutylphenyl. Such pyrrolopyrrole derivatives provide excellent rheology improvement properties when incorporated into a pigment composition, but additionally provide enhancement of desirable pigment properties such as hiding (opacity) and color saturation. Thus, the pyrrolopyrrole derivative wherein A₁, A₂, A₃ and A₄ are each 4-tertbutylphenyl represents an especially preferred compound of the present invention.

Another preferred class of pyrrolopyrrole derivatives are those wherein A₃ and A₄ are each 4-methylphenyl; and A₁ and A₂ are identical and selected from the group consisting of phenyl, 4-tertbutylphenyl and 4-methylphenyl; especially 4-tertbutylphenyl.

The pyrrolopyrrole derivatives of the present invention are especially suitable for use as rheology-improving agents for organic pigments. As rheology-improving agents for organic pigments, the inventive pyrrolopyrrole derivatives function to reduce the viscosity of a dispersion of the pigment composition in a high-molecular-weight organic material, such as a coating composition, for example, a water-borne or solvent-borne automotive paint.

Thus, another aspect of this invention relates to pigment compositions which comprise an organic pigment and a pyrrolopyrrole derivative of formulae (I, Ia and/or Ib). Preferably, the pigment composition contains from 0.1 to 20 percent by weight of the pyrrolopyrrole derivative. Most preferably, the pigment composition contains from 1 to 10 percent by weight of the pyrrolopyrrole derivative.

Preferably, the pyrrolopyrrole derivative of formulae (I, Ia and/or Ib) is selected such that A₁, A₂, A₃ and A₄ are each a group of formula 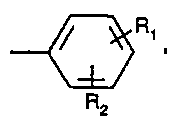 wherein R₁ and R₂ are each independently of the other hydrogen, methyl, tert-butyl, chloro, bromo, phenyl or CN. At least one of R₁ and R₂ is preferably hydrogen. Most preferably, at least one of R₁ and R₂ is hydrogen and the other is in the 3- or 4-position of the phenyl ring.

Important pigment compositions according to the present invention are those which contain a 1,4-diketo-3,6-diarylpyrrolo[3,4-c]pyrrole pigment or a quinacridone pigment as the organic pigment including their solid solutions and a pyrrolopyrrole derivative of the formulae (I, Ia and/or Ib).

The inventive pigment compositions especially include those containing a 1,4-diketo-3,6-diarylpyrrolo[3,4-c]pyrrole pigment selected from the group consisting of unsubstituted 1,4-diketo-3,6-diphenylpyrrolo[3,4-c]pyrrole, 1,4-diketo-3,6-di(3- or 4-chlorophenyl)pyrrolo[3,4-c]pyrrole, 1,4-diketo-3,6-di(3,4-dichlorophenyl)pyrrolo[3,4-c]pyrrole, 1,4-diketo-3,6-di(3-cyanophenyl)pyrrolo[3,4-c]pyrrole, 1,4-diketo-3,6-di(4-tert-butylphenyl)pyrrolo[3,4-c]pyrrole, 1,4-diketo-3,6-di(4-methylphenyl)pyrrolo[3,4-c]pyrrole, and 1,4-diketo-3,6-di(biphenyl-1-yl)pyrrolo[3,4-c]pyrrole, and a pyrrolopyrrole derivative of formulae (I, Ia and/or lb).

In addition, the inventive pigment compositions especially include those containing a quinacridone pigment selected from the group consisting of unsubstituted quinacridone, 2,9- and 4,11-dimethylquinacridone, 2,9- and 4,11-dichloroquinacridone and 2,9- and 4,11-difluoroquinacridone and a pyrrolopyrrole derivative of formulae (I, Ia and/or Ib).

Especially important pigment compositions are those containing a 1,4-diketo-3,6-diarylpyrrolo[3,4-c]pyrrole pigment selected from the group consisting of unsubstituted 1,4-diketo-3,6-diphenylpyrrolo[3,4-c]pyrrole and 1,4-diketo-3,6-di(4-tert-butylphenyl)pyrrolo[3,4-c]pyrrole in combination with a pyrrolopyrrole derivative of formulae (I,and/or Ia), and/or of formula (Ib) wherein A₃ and A₄ are each 4-tertbutylphenyl, especially those wherein in addition A₁ and A₂ are identical and selected from phenyl, 4-tert.butylphenyl and 4-methylphenyl; especially 4-tert.butylphenyl, most preferred, if the organic pigment is red or orange colored, or wherein A₃ and A₄ are each 4-methylphenyl, especially those wherein in addition A₁ and A₂ are identical and selected from phenyl, 4-tert.butylphenyl and 4-methylphenyl; especially 4-tert.butylphenyl.

Although the pigment compositions of the present invention can consist of only the organic pigment and the pyrrolopyrrole derivative, the pigment compositions generally contain customary additives, such as texture improving agents, light stabilizers and especially a second rheology-improving agent. In this application, the expression "pigment composition" preferably means a formulation which is to be dispersed in a high-molecular-weight organic material that is composed of the pigment, the pyrrolopyrrole derivatives and any optional additives.

Useful light stabilizers are U.V. light absorbers, for example, benzotriazoles or hindered amine light stabilizers (HALS).

Texture-improving agents are especially useful as an additional component which can improve the properties of the stir-in pigment compositions. Suitable texture-improving agents include fatty acids having at least 12 carbon atoms, and amides, esters or salts of fatty acids. Typical fatty acid derived texture-improving agents include fatty acids such as stearic acid or behenic acid, and fatty amines such as lauryl amine, or stearylamine. In addition, polyols, such as aliphatic 1,2-diols or polyvinyl alcohol, and ethoxylated fatty alcohols, epoxidized soya bean oil, waxes, resin acids and resin acid salts are suitable texture-improving agents. Rosin acids and rosin acid salts are especially suitable texture-improving agents. In general, the inventive pigment compositions contain from 0 to 20 percent by weight of the texture improving agent, preferably 0.5 to 10 percent by weight.

Agents useful as the second rheology-improving agent in the present pigment compositions include quinacridone derivatives, such as, quinacridone sulfonic acid, or a salt thereof, especially the aluminum salt, or pyrazolylmethylquinacridone, or other pyrrolopyrrole (DPP) derivatives, such as, a DPP sulfonic acid, or a salt thereof, or a another DPP derivative of the formula I. If the pigment composition includes a second rheology-improving agent, the combined parts by weight of the pyrrolopyrrole derivative of formulae (I, Ia and/or Ib) and the second rheology-improving agent are preferably in the range from 0.2 to 20 parts by weight per part of the pigment; most preferably from 2 to 10 parts per part of the pigment.

As discussed above, the presence of an inventive pyrrolopyrrole derivative in a dispersion of an organic pigment in a high-molecular-weight organic material effectively reduces the viscosity of the dispersion. Thus, the present invention further relates to a method of reducing the viscosity of a dispersion of an organic pigment in a high-molecular-weight organic material which comprises incorporating an effective viscosity-reducing amount of a pyrrolopyrrole derivative of formulae (I, Ia and/or Ib) into the dispersion.

The pyrrolopyrrole derivative of formulae (I, Ia and/or Ib) is added to the dispersion on its own, or, preferably, as a component of a pigment composition.

Preferably, the pyrrolopyrrole derivative of formula (Ib) is chosen such that A₁, A₂, A₃ and A₄ are each a group of formula 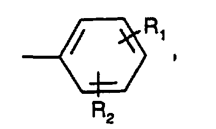 wherein R₁ and R₂ are each independently of the other hydrogen, methyl, tert-butyl, chloro, bromo, phenyl or CN. At least one of R₁ and R₂ is preferably hydrogen. Most preferably, at least one of R₁ and R₂ is hydrogen and the other is in the 3- or 4-position of the phenyl ring.

Preferably, the pyrrolopyrrole derivative of formulae (I, Ia and/or Ib) is present in the dispersion in an amount in the range from 0.1 to 20 parts by weight per part of the pigment dispersion. Preferably, the pyrrolpyrrole derivative of formulae (I, Ia and/or Ib) is present in the dispersion in an amount in the range from 0.2 to 10 parts by weight per part of the pigment dispersion. If the pyrrolopyrrole derivative of formulae (I, Ia and/or Ib) is combined with a second rheology-improving agent, the combined parts by weight of the pyrrolopyrrole derivative of formulae (I, Ia and/or Ib) and the second rheology-improving agent are preferably in the range from 0.1 to 20 parts by weight per part of the pigment dispersion; most preferably from 0.2 to 10 parts per part of the pigment dispersion.

The organic pigment is an azo, azomethine, anthraquinone, phthalocyanine, perinone, perylene, 1,4-diketo-3,6-diarylpyrrolo[3,4-c]pyrrole, thioindigo, isoindoline, isoindolinone, quinacridone, flavanthrone, indanthrone, anthrapyrimidine and quinophthalone pigments; in particular a 1,4-diketo-3,6-diarylpyrrolo[3,4-c]pyrrole or quinacridone pigment, or a mixture thereof.

The high-molecular-weight organic materials are, for example, cellulose ethers, cellulose esters, polyurethanes, polyesters, polycarbonates, polyolefins, polystyrene, polysulfones, polyamides, polycycloamides, polyimides, polyethers, polyether ketones, polyvinyl halides, polytetrafluoroethylene, acrylic and methacrylic polymers, rubber, silicone polymers, phenol/formaldehyde resins, melamine, formaldehyde resins, urea/formaldehyde resins, epoxy resins and diene rubbers or copolymers thereof.

The dispersions especially contain a high-molecular-weight organic material which is useful for heat-curable or cross-linked coatings, for example chemically-reactive coatings, including stoving finishes which contain the customary binders and which are reactive at high temperature. Examples of the high-molecular-weight organic materials which are used in such coatings include acrylic, alkyd, epoxy, phenolic, melamine, urea, polyester, polyurethane, blocked isocyanate, benzoguanamine or cellulose ester resins, or combinations thereof. The pigmented, high-molecular-weight organic materials prepared according to the present process are also useful as air-drying or physically-drying coatings, for example, conventional lacquers such as those used in the cosmetics industry as nail varnishes, for example nitrocellulose lacquers.

The present process is particularly suitable for reducing the viscosity of dispersions containing high-molecular-weight organic materials conventionally employed as finishes in the automobile industry, especially acrylic/melamine resin, alkyd/melamine resin or thermoplastic acrylic resin systems, as well as in aqueous-based coating systems.

The use of many of the known viscosity-reducing agents for pigment dispersions often results in at least some loss of saturation when the pigment dispersion is applied as the basecoat in a basecoat/clearcoat automotive finish. However, the use of the present pyrrolopyrrole derivatives of formulae (I, Ia and/or Ib) as viscosity-reducing agents suprisingly does not result in loss of saturation when the pigment dispersion is applied as the basecoat in a basecoat/clearcoat automotive finish, indeed in many instances the saturation of the finish is increased by the presence of the pyrrolopyrrole derivative of formulae (I, Ia and/or Ib).

Thus, the present invention further relates to a method of preparing a basecoat/clearcoat finish, which comprises the step of applying a dispersion comprising an effective viscosity-reducing and saturation-enhancing amount of a pyrrolopyrrole derivative of formulae (I, Ia and/or Ib) in a high-molecular-weight organic compound to a substrate; especially wherein in the compound of formula (Ib) A₃ and A₄ are each 4-tert-butylphenyl; and A₁ and A₂ are identical and selected from the group consisting of phenyl, 4-tertbutylphenyl and 4-methylphenyl; especially 4-tert-butylphenyl.

Another preferred embodiment relates to a method of preparing an basecoat/clearcoat finish, which comprises the step of applying a dispersion comprising an organic pigment and an effective viscosity-reducing amount of a pyrrolopyrrole derivative of formulas (I, Ia, Ib) in a high-molecular-weight organic compound to a substrate.

A preferred embodiment of the present invention relates to a coating composition, such as an automotive finish, which comprises a high-molecular-weight organic material, a red- or orange-colored organic pigment and a pyrrolopyrrole derivative of the formula (Ib) wherein A₃ and A₄ are each 4-tertbutylphenyl; especially those wherein A₁ and A₂ are identical and selected from phenyl, 4-tertbutylphenyl and 4-methylphenyl; especially phenyl or 4-tert.-butylphenyl.

In addition, a preferred embodiment of this invention includes a coating composition, which comprises a high-molecular-weight organic material, a red- or orange-colored organic pigment and a pyrrolopyrrole derivative of the formula (Ib) wherein A₁, A₂, A₃ and A₄ are each phenyl, or A₁ and A₂ are phenyl and A₃ and A₄ are each 4-tert.-butylphenyl.

Preferably, the red- or orange-colored organic pigment is selected from the group consisting of unsubstituted 1,4-diketo-3,6-diphenylpyrrolo[3,4-c]pyrrole and 1,4-diketo-3,6-di(4-tert.-butylphenyl)pyrrolo[3,4-c]pyrrole.

Preferably, the high-molecular-weight organic material is an acrylic/melamine resin, alkyd/melamine resin or thermoplastic acrylic resin.

Preferences discussed above for the pyrrolopyrrole derivatives relate to all other aspects of this invention.

In this application, the expression "automotive finish" is used to describe finishes typically used for automobiles, such as waterborne and solventborne basecoat/clearcoat finishes. Such finishes are used for numerous applications other than for automobiles, such as other motor vehicles, bicycles, and appliances.

In addition to being useful as an intermediate to prepare the diketopyrrolopyrrole derivatives of formulae (I, Ia and/or Ib), the hydroxymethyl pyrrolopyrrole compound of formula (II) is useful as a stabilizer for polymers.

The diketopyrrolopyrrole derivatives of formulae (I, Ia and Ib) are useful as colorants, such as pigments and dyes, for a variety of materials, in particular paints, plastics and inks, and as additives which influence the rheology characteristics of a pigment composition.

In general, diketopyrrolopyrrole derivatives of formulae (I, Ia and Ib) with a lower degree of sulfonation are most suitable as pigments, while those with a higher degree of sulfonation are useful as dyes and additives.

The following examples further illustrate the preferred embodiments of this invention. In these examples, all parts given are by weight unless otherwise noted All viscosity measurements are taken at room temperature.

Example 1: 500 grams of concentrated sulfuric acid (95.28%) are added to a one liter four-necked flask equipped with a stirrer, a thermometer and a reflux condenser with a drying tube. 6.2 grams (0.206 moles) of paraformaldehyde are then introduced into the sulfuric acid followed by 28.8 grams (0.1 moles) of 1,4-diketo-3,6-diphenylpyrrolo[3,4-c]pyrrole (unsubstituted DPP) in small portions maintaining the pot temperature below 30°C. The reaction mixture is stirred at 25±2°C for 2.5 hours and then poured into ice water, filtered, washed with water until the filtrate is acid free, dried and pulverized to yield 2,5-di(hydroxymethyl)-3,6-diphenylpyrrolo[3,4-c]pyrrole-1,4-dione (analysis C₂₀H₁₆N₂O₄).

Examples 2-5: The bis(hydroxymethyl) DPP compounds disclosed in Table 1 are prepared by substituting the appropriate substituted DPP for unsubstituted DPP in the process of Example 1:

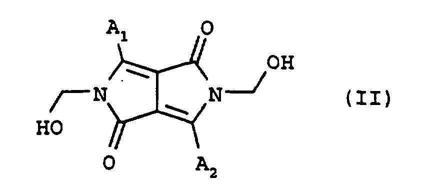

**Table-1**

| Bishydroxymethyl DPP derivatives | | | | |
|---|---|---|---|---|
| A1 | A2 | Mol. Form | Mol. Wt | crystallized from |
| 4-CI-phenyl | 4-CI-phenyl | C₂₀H₁₄Cl₂N₂O₄ | 417 | DMF-MeOH |
| p-tolyl | p-tolyl | C₂₂H₂₀N₂O₄ | 376 | DMF-MeOH |
| 4-t-butyl-phenyl | t-butyl-phenyl | C₂₈H₃₂N₂O₄ | 460 | DMF-MeOH |
| biphenyl-1-yl | biphenyl-1-yl | C₃₂H₂₄N₂O₄ | 500 | DMF-MeOH |

Example 6: 250 grams of concentrated sulfuric acid (96%) are added to a one liter four-necked flask equipped with a stirrer, a thermometer and a reflux condenser with a drying tube. 14.4 grams (0.05 moles) of 1,4-diketo-3,6-diphenylpyrrolo[3,4-c]pyrrole (unsubstituted DPP) are added in small portions maintaining the pot temperature below 25°C. 3.1 grams (0.103 moles) of paraformaldehyde are then introduced into the reaction mixture. The reaction mixture is stirred at 25±2°C for four hours and then poured into ice water, filtered, washed with water until the filtrate is acid free, dried and pulverized to yield 2,5-di(hydroxymethyl)-3,6-diphenylpyrrolo[3,4-c]pyrrole-1,4-dione (analysis C₂₀H₁₆N₂O₄).

Example 7: 401.2 grams of concentrated sulfuric acid (96%) are added to a one liter four-necked flask equipped with a stirrer, a thermometer and a reflux condenser with a drying tube. 17.85 grams (0.05 moles) of 1,4-diketo-3,6-di(4-chlorophenyl)pyrrolo[3,4-c]pyrrole (dichloro DPP) are added in small portions maintaining the pot temperature below 30°C. 3.1 grams (0.103 moles) of paraformaldehyde are then introduced into the reaction mixture. The reaction mixture is stirred at 25±2°C for four hours and then poured into ice water, filtered, washed with water until the filtrate is acid free, dried and pulverized to yield 2,5-di(hydroxymethyl)-3,6-di(4-chlorophenyl)pyrrolo[3,4-c]pyrrole-1,4-dione (analysis C₂₀H₁₄Cl₂N₂O₄).

Example 8: 422.9 grams of concentrated sulfuric acid (96%) are added to a one liter four-necked flask equipped with a stirrer, a thermometer and a reflux condenser with a drying tube. 22.0 grams (0.05 moles) of 1,4-diketo-3,6-di(biphenyl-1-yl)pyrrolo[3,4-c]pyrrole (biphenylyl DPP) are added in small portions maintaining the pot temperature below 30°C. 3.1 grams (0.103 moles) of paraformaldehyde are then introduced into the reaction mixture. The reaction mixture is stirred at 25±2°C for four hours and then poured into ice water, filtered, washed with water until the filtrate is acid free, dried and pulverized to yield 2,5-di(hydroxymethyl)-3,6-di(biphenyl-1-yl)pyrrolo[3,4-c]pyrrole-1,4-dione (analysis C₃₂H₂₄N₂O₄).

Example 9: 250 grams of concentrated sulfuric acid (96%) are added to a one liter four-necked flask equipped with a stirrer, a thermometer and a reflux condenser with a drying tube. Quinacridone (31.2 grams, 0.1 moles) is added in small portions followed by 14.4 grams (0.05 moles) of 1,4-diketo-3,6-diphenylpyrrolo[3,4-c]pyrrole (unsubstituted DPP) maintaining the pot temperature between 40-45°C. After stirring for 0.5 hours, 3.1 grams (0.103 moles) of paraformaldehyde are then introduced into the reaction mixture and the temperature rises to about 50°C. The reaction is stirred at 45±3°C for 1 hour and then poured into ice water, filtered, washed with water until the filtrate is acid free, dried and pulverized to yield the compound of the formula 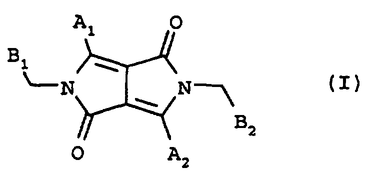 wherein A₁ and A₂ are each phenyl and B₁ and B₂ are quinacridinyl radicals. The product analyzes C₆₀H_{35.5}N₆O₆ • (SO₃H)_{0.5}.

Example 10: 250 grams of concentrated sulfuric acid (96%) are added to a one liter four-necked flask equipped with a stirrer, a thermometer and a reflux condenser with a drying tube. 14.4 grams (0:05 moles) of 1,4-diketo-3,6-diphenylpyrrolo[3,4-c]pyrrole (unsubstituted DPP) are then added in small portions maintaining the pot temperature below 40°C. After stirring for 0.5 hours, 3.1 grams (0.103 moles) of paraformaldehyde are then introduced into the reaction mixture and the temperature rises to about 45°C. The reaction is stirred at 40±2°C for 1 hour. 31.2 grams of quinacridone (0.1 moles) of quinacridone is then added maintaining the temperature below 45°C. The reaction mixture is stirred at 45±2°C for 3 hours and then poured into ice water, filtered, washed with water until the filtrate is acid free, dried and pulverized to yield the compound of the formula 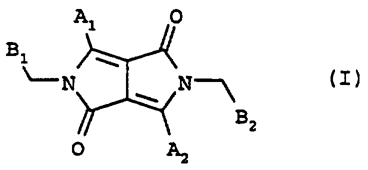 wherein B₁ and B₂ are quinacridinyl radicals. The product analyzes C₆₀H_{35.5}N₆O₆ • (SO₃H)_{0.5}.

Example 11: 250 grams of concentrated sulfuric acid (96%) are added to a one liter four-necked flask equipped with a stirrer, a thermometer and a reflux condenser with a drying tube. 14.4 grams (0.05 moles) of 1,4-diketo-3,6-diphenylpyrrolo[3,4-c]pyrrole (unsubstituted DPP) are then added in small portions maintaining the pot temperature below 25°C. After stirring for 1 hour, 3.1 grams (0.103 moles) of paraformaldehyde are then introduced into the reaction mixture followed by 31.2 grams of quinacridone (0.1 moles) and the maintaining the temperature below 25°C. The reaction mixture is stirred at 25±2°C for 3 hours and then poured into ice water, filtered, washed with water until the filtrate is acid free, dried and pulverized to yield the compound of the formula 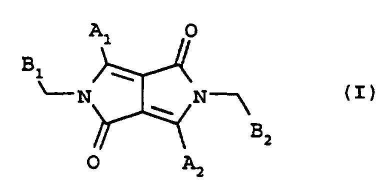 wherein B₁ and B₂ are quinacridinyl radicals. The product analyzes C₆₀H₃₆N₆O₆.

Example 12: 400 grams of concentrated sulfuric acid (96%) are added to a one liter four-necked flask equipped with a stirrer, a thermometer and a reflux condenser with a drying tube. 14.4 grams (0.05 moles) of 1,4-diketo-3,6-diphenylpyrrolo(3,4-c]pyrrole (unsubstituted DPP) are then added in small portions maintaining the pot temperature below 25°C. After stirring for 1 hour, 3.1 grams (0.103 moles) of paraformaldehyde are then introduced into the reaction mixture followed by 38.1 grams of 4,11-dichloroquinacridone (0.1 moles) and the maintaining the temperature below 45°C. The reaction mixture is stirred at 45±2°C for 2.5 hours and then poured into ice water, filtered, washed with water until the filtrate is acid free, dried and pulverized to yield the compound of the formula 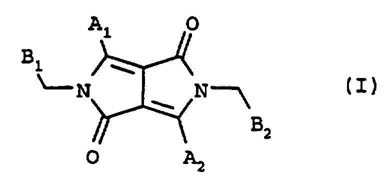 wherein B₁ and B₂ are 4,11-dichloroquinacridinyl radicals. The product analyzes C₆₀H₃₄C₁₂N₆O₆.

Example 13: 500 grams of concentrated sulfuric acid (90%) are added to a one liter four-necked flask equipped with a stirrer, a thermometer and a reflux condenser with a drying tube. 6.2 grams (0.206 moles) of paraformaldehyde are then introduced into the sulfuric acid followed by 28.8 grams (0.1 moles) of 1,4-diketo-3,6-diphenylpyrrolo[3,4-c]pyrrole (unsubstituted DPP) in small portions maintaining the pot temperature below 30°C. The reaction mixture is stirred at 25±2°C for 2.5 hours and then poured into ice water, filtered, washed with water until the filtrate is acid free, dried and pulverized to yield a mixture of 2,5-di(hydroxymethyl)-3,6-diphenylpyrrolo[3,4-c]pyrrole-1,4-dione and the starting materials. When 85% H₂SO₄ replaces the 90% H₂SO₄, a mixture containing a major portion of the starting materials is obtained.

Example 14: 250 grams of concentrated sulfuric acid (96%) and paraformaldehyde (3.3g.; 0.11 moles) are added to a one liter four necked flask, equipped with a stirrer, a thermometer, a reflux condenser with a drying tube. Unsubstituted DPP(14.4g.; 0.05moles) are added to the sulfuric acid/paraformaldehyde mixture in small portions maintaining the pot temperature between 25-30 °C. This mixture is stirred for 1 hour to ensure complete solution. An additional 200g of sulfuric acid is then added to the reaction mixture followed by unsubstituted DPP(28.8g.; 0.1 moles) in small portions, maintaining the pot temperature at 32-34°C. This reaction mixture is then stirred at 30±3°C for 4 hrs and poured into ice-water, filtered, washed with water until the filtrate is acid free, dried and pulverized.

The compound isolated analyzed for C₅₆H₃₆N₆O₆ and is believed to have the formula IV 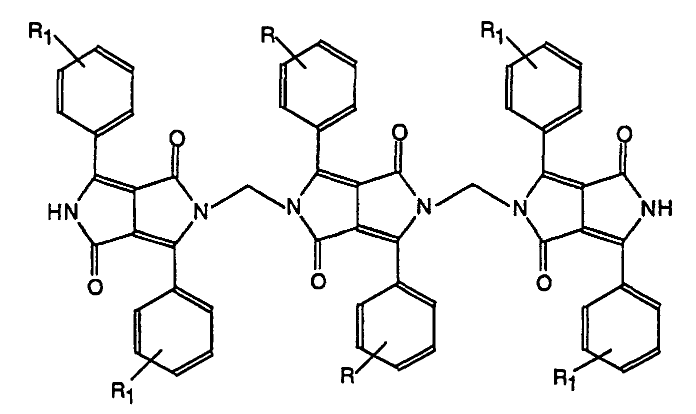 wherein each R and R₁ substituent is hydrogen.

The compounds in Table 2 are made by substituting the appropriate pyrrolopyrrole reactants in the process of Example 14.

**TABLE 2**

| DPP-CH₂-DPP-CH₂-DPP derivatives | | | |
|---|---|---|---|
| R | R₁ | Mol. Form | crystallized from |
| H | H | C₅₆H₃₆N₆O₆ | H₂SO₄ |
| H | Cl | C₅₆H₃₂Cl₄N₆O₆ | H₂SO₄ |
| H | CH₃ | C₆₀H₄₄N₆O₆ | H₂SO₄ |
| H | t-Butyl | C₇₂H₆₈N₆O₆ | H₂SO₄ |
| H | 4-Phenyl | C₈₀H₅₂N₆O₆ | H₂SO₄ |
| Cl | H | C₅₆H₃₄Cl₂N₆O₆ | H₂SO₄ |
| Cl | Cl | C₅₆H₃₀Cl₆N₆O₆ | H₂SO₄ |
| Cl | CH₃ | C₆₀H₄₂Cl₂N₆O₆ | H₂SO₄ |
| Cl | t-Butyl | C₇₂H₆₆Cl₂N₆O₆ | H₂SO₄ |
| Cl | 4-Phenyl | C₈₀H₅₀Cl₂N₆O₆ | H₂SO₄ |
| CH3 | H | C₅₈H₄₀N₆O₆ | H₂SO₄ |
| CH₃ | Cl | C₅₈H₃₆Cl₄N₆O₆ | H₂SO₄ |
| CH₃ | CH₃ | C₆₂H₄₈N₆O₆ | H₂SO₄ |
| CH₃ | t-Butyl | C₇₄H₇₂N₆O₈ | H₂SO₄ |
| CH₃ | 4-Phenyl | C₈₂H₅₆N₆O₆ | H₂SO₄ |
| t-Butyl | H | C₆₄H₅₂N₆O₆ | H₂SO₄ |
| t-Butyl | Cl | C₆₄H₄₈Cl₄N₆O₆ | H₂SO₄ |
| t-Butyl | CH₃ | C₆₈H₆₀N₆O₆ | H₂SO₄ |
| t-Butyl | t-Butyl | C₈₀H₈₄N₆O₆ | H₂SO₄ |
| t-Butyl | 4-Phenyl | C₈₈H₆₈N₆O₆ | H₂SO₄ |
| 4-Phenyl | H | C₆₈H₄₄N₆O₆ | H₂SO₄ |
| 4-Phenyl | Cl | C₆₈H₄₀Cl₄N₆O₆ | H₂SO₄ |
| 4-Phenyl | CH₃ | C₇₂H₅₂N₆O₆ | H₂SO₄ |
| 4-Phenyl | t-Butyl | C₈₄H₇₆N₆O₆ | H₂SO₄ |
| 4-Phenyl | 4-Phenyl | C₉₂H₆₀N₆O₆ | H₂SO₄ |

Example 15: 250 grams of concentrated sulfuric acid (96%) and paraformaldehyde (3.3g.; 0.11 moles) are added to a one liter four necked flask, equipped with a stirrer, a thermometer, a reflux condenser with a drying tube. Unsubstituted DPP(14.4g.; 0.05moles) are added to the sulfuric acid/paraformaldehyde mixture in small portions maintaining the pot temperature between 30-35 °C. This mixture is stirred for 1 hour to ensure complete solution. An additional 200g of sulfuric acid is then added to the reaction mixture followed by unsubstituted DPP(28.8g.; 0.1 moles) in small portions, maintaining the pot temperature at 38-42 °C. This reaction mixture is then stirred at 40±3°C for 4 hrs and poured into ice-water, filtered, washed with water until the filtrate is acid free, dried and pulverized. The compound isolated analyzed for C₅₆H₃₆N₆O₆·(SO₃H)_{0.5}

The sulfonated derivatives of the compounds of Table 2 are prepared by substituting the appropriate pyrrolopyrrole reactants in the process of Example 15.

Example 16: Bishydroxymethyl DPP (17.4g, 0.05 moles), aniline (9.3g, 0.1 moles) and DMF (100ml) are added to a one liter four necked flask, equipped with a stirrer, a thermometer, and a reflux condenser with a drying tube. The mixture is stirred at 100°C for 4 hrs, then diluted with water, filtered, washed with water, dried and pulverized to give 2,5-di(anilinomethyl)-3,6-diphenyl pyrrolo[3,4-c]pyrrole-1,4-dione (bisanilinomethyl DPP).

Example 17: Bishydroxymethyl DPP (17.4g, 0.05 moles), a catalytic amount of paratoluene sulfonic acid and methanol (100ml) are added to a one liter four necked flask, equipped with a stirrer, a thermometer, and a reflux condenser with a drying tube. The mixture is stirred at reflux for 4 hrs., diluted with water, filtered, washed with water, dried and pulverized to give 2,5-di(methoxymethyl)-3,6-diphenyl pyrrolo[3,4-c]pyrrole-1,4-dione (bismethoxymethyl DPP).

Example 18: Bishydroxymethyl-di-t-butyl DPP (23.0g, 0.05 moles), a catalytic amount of paratoluene sulfonic acid and methanol (100ml) are added to a one liter four necked flask, equipped with a stirrer, a thermometer, and a reflux condenser with a drying tube. The mixture is stirred at reflux for 4 hrs, diluted with water, filtered, washed with water, dried and pulverized to give 2,5-di(methoxymethyl)-3,6-di-t-butylphenyl pyrrolo[3,4-c]pyrrole-1,4-dione (bismethoxymethyl di-t-butyl DPP).

Examples 19-26: The following compounds, wherein A₁, A₂ and R are defined according to formulae (la) and (III), are prepared according to the procedures of Examples 9-12 by substituting the appropriate DPP and quinacridone compounds:

| Ex | A₁ | A₂ | R |
|---|---|---|---|
| 19 | phenyl | phenyl | 2,9-diCl |
| 20 | phenyl | phenyl | 2,9-diMe |
| 21 | phenyl | phenyl | 4,11-diMe |
| 22 | 4-tbu-phenyl | 4-tbu-phenyl | 2,9-diCl |
| 23 | 4-CI-phenyl | 4-CI-phenyl | H |
| 24 | 4-Me-phenyl | 4-Me-phenyl | H |
| 25 | 4-tbu-phenyl | 4-tbu-phenyl | H |
| 26 | 4-Me-phenyl | 4-Me-phenyl | 2,9-diCl |

Example 27: A pigment composition is prepared by adding a surface treating agent which consists of the pyrrolopyrrole derivative of formula (la), alone, or combined with a second rheology-improving agent, to an aqueous suspension of the pigment, filtering and washing to yield the pigment composition. The viscosity of a dispersion of the pigment composition in a commercial high solids solvent-borne paint formulation is measured at 50 rpm.

| Pyrrolopyrrole Derivative | Second Rheology Improving Agent | Pigment | % by Weight Pigment in Dispersion | P/B | Millbase Viscosity (Brookfield) |
|---|---|---|---|---|---|
| none | none | 1 | 10 | 0.5 | 1610 |
| 8% Example 11 | none | 1 | 10 | 0.5 | 63 |
| 4% Example 11 | 4%QMA | 1 | 10 | 0.5 | 62 |
| 4% Example 24 | 4% PMQA | 2 | 10 | 0.5 | 600 |
| 4% Example 19 | 4%PMQA | 2 | 10 | 0.5 | 708 |
| 4% Example 22 | 4% PMQA | 2 | 10 | 0.5 | 648 |
| 4% Example 25 | 4% PMQA | 2 | 10 | 0.5 | 474 |
| 4% Example 10 | 4% QMA | 2 | 10 | 0.5 | 736 |
| none | none | 3 | 10 | 0.5 | 1910 |
| 4% Example 10 | 4% QMA | 3 | 10 | 0.5 | 1210 |
| QMA=quinacridone sulfonic acid, aluminum salt | | | | | |
| PMQA=pyrrazolylmethylquinacridone | | | | | |
| 1=a ternary solid solution of dichloro DPP/DPP/2,9-dichloroQA (48/32/20) | | | | | |
| 2=binary solid solution of dichloro DPP/2,9-dichloroQA(60/40) | | | | | |
| 3=1,4-diketo-3,6-di(biphenyl-1-yl)pyrrolopyrrole | | | | | |

Example 28: A solid solution of dichloro DPP/dichloroquinacridone (40/60) is prepared by milling a mixture of the two individual components on a roller for 24 hours and stirring the mill powder with 2% sulfuric acid for 2 hours at 90°C, and then filtering and washing the resulting pigment slurry. The resulting presscake is reslurried in water and combined with the rheology-improving agent(s) specified in the table below, filtered, washed, dried and pulverized to yield a treated pigment. A pigment dispersion containing 10% pigment and 30% solids at a pigment/binder ratio of 0.5 is prepared by combining the treated pigment with acrylourethane resin, dispersant resin and solvent and milling with grinding media for 64 hours. The Brookfield viscosity of the resulting pigment dispersion is reported in the following table.

| Pyrrolopyrrole Derivative | Second Rheology Improving Agent | Millbase Viscosity (Brookfield) |
|---|---|---|
| 4% Example 10 | 4% PQMA | 566 |
| 4% Example 24 | 4% PQMA | 600 |
| 4% Example 25 | 4% PQMA | 474 |
| 4% Example 26 | 4% PQMA | 708 |
| 4% Example 22 | 4% PQMA | 648 |

Example 29: Automotive finishes are obtained by spray painting aluminum panels with a metallic or mica paint formulation prepared by conventional methods and pigmented with the above described pigment compositions. The coatings exhibit attractive, intense colors with excellent two-tone, high gloss and distinctness of image.

Example 30: An organic ternary solid solution pigment which is composed of dichloro DPP/unsubstituted DPP / 2,9-dichloroquinacridone (48% / 32% /20%), is combined with a rheology-improving agent composed of 4% quinacridone monosulfonic acid, aluminum salt (QMA) and 4% of the pyrrolopyrrole derivative of Example 14 by adding the rheology-improving agent to an aqueous suspension of the pigment, filtering and washing to yield the pigment composition. Rheology profiles of pigment dispersions in a commercial high solids solvent-borne paint formulation containing about 12% by weight of the pigment at a pigment to binder ratio of 0.5% are depicted in the Table 3.

**Table 3**

| MILLBASE VISCOSITY (BROOKFIELD) | | |
|---|---|---|
| Paint System: BC / CC; % Pigment: 12.0 %; P / B = 0.5 | | |

| Sample ID | Rheology-improving Agent | 50 RPM |
|---|---|---|
| 30a | Untreated | 3660 |
| 30b | 4%QMA+4% Example 14 R=R₁=H | 828 |

Example 31: An orange-colored di(t-butylphenyl)-DPP pigment is combined with a rheology-improving agent described according to formula (IV) given in ex. 14. Rheology profiles of pigment dispersions in a commercial high solids solventborne paint formulation containing about 16% by weight of the pigment at a pigment to binder ratio of 0.5% measured after 4 hours of attritor milling are depicted in the Table 4.

**Table 4**

| Millbase Viscosity (Brookfield) | | |
|---|---|---|
| sample | Rheology-improving Agent | 50 rpm |
| | | |
| 31a | 4% R=H; R₁=CH₃ | 868 |
| 31b | 8% R=H; R₁=CH₃ | 546 |
| 31c | 4% R=t-Bu, R₁=t-Bu | 670 |
| 31d | 8% R=t-Bu, R₁=t-Bu | 528 |

The rheology-improving of examples 31c and 31d, in addition to the rheology improvements, improves the color saturation of the pigment when sprayed on a panel as part of a conventional basecoat/clearcoat automotive finish. Similarly improved color saturation is observed when a different red or orange pigment, for example quinacridone, is used in place of di(t-butylphenyl)-DPP.

Example 32: An orange-colored di(t-butylphenyl)-DPP pigment is combined with the mixture of rheology-improving agents described in the Table 5 with R and R₁ being described according to formula (IV) given in ex. 14 and PYMQ being pyrrazolylmethylquinacridone. Rheology profiles of pigment dispersions in a commercial high solids solventborne paint formulation containing about 16% by weight of the pigment at a pigment to binder ratio of 0.5% measured after 4 hours of attritor milling are depicted in the Table 5.

**Table 5**

| Millbase Viscosity (Brookfield) | | |
|---|---|---|
| Sample | Rheology-improving Agent | 50rpm |
| 32 | no treatment | >2000 |
| 32a | 4%PYMQ + 4% R=H; R₁=CH₃ | 954 |
| 32b | 4% R=H, R₁=tBu + 4% R=H; R₁=CH₃ | 1080 |
| 32c | 2% R=H, R₁=tBu + 2% R=H; R₁ = CH₃ | 1320 |

## Claims

1. A process for the preparation of a diketopyrrolopyrrole derivative of the formula (I) 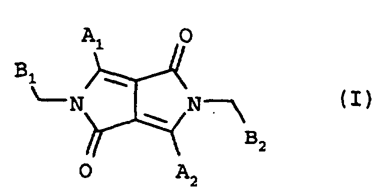 wherein A₁ and A₂ are radicals of the formula 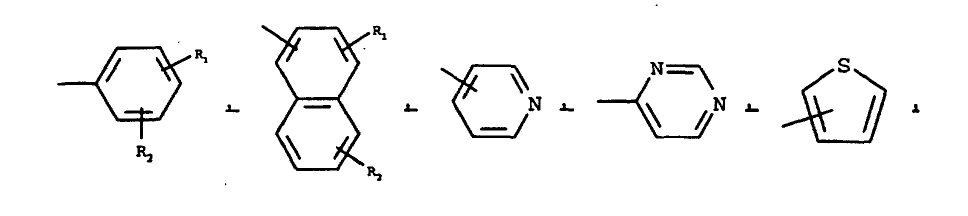 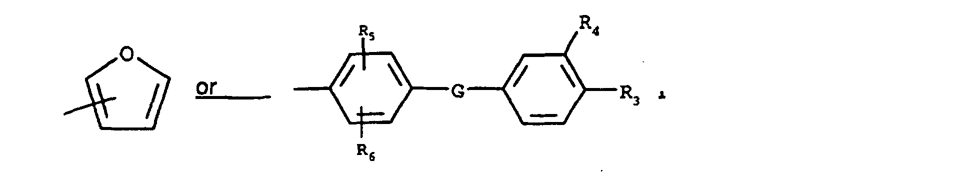 wherein
R₁ and R₂ are each independently of the other hydrogen, halogen, C₁-C₁₈alkyl, C₁-C₁₈alkoxy, C₁-C₁₈alkylmercapto, C₁-C₁₈alkylamino, C₁-C₁₈alkoxycarbonyl, C₁-C₁₈alkylaminocarbonyl, -CN, -NO₂, trifluoromethyl, C₅-C₆cycloalkyl, -C=N-(C₁-C₁₈alkyl), phenyl, 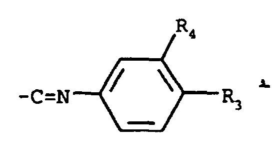 imidazolyl, pyrrazolyl, triazolyl, piperazinyl, pyrrolyl, oxazolyl, benzoxazolyl, benzothiazolyl, benzimidazolyl, morpholinyl, piperidinyl or pyrrolidinyl,
G is -CH₂-, -CH(CH₃)-, -C(CH₃)₂-, -CH=N-, -N=N-, -O-, -S-, -SO-, -SO₂-, -CONH- or -NR₇-,
R₃ and R₄ are each independently of the other hydrogen, halogen, C₁-C₆alkyl, C₁-C₁₈alkoxy or -CN, R₅ and R₆ are each indenendently of the other hydrogen, halogen or C₁-C₆alkyl, and R₇ is hydrogen or C₁-C₆alkyl, and B₁ and B₂ are identical or different organic radicals which derive from a precursor which reacts with the hydroxy groups of a compound of formula (II) either by substitution reaction or to form an -O-linkage; wherein the precursor which reacts with a hydroxyl group by a substitution reaction is a compound which is selected from the group consisting of an aromatic radical, a heteroaromatic radical, or radicals of the formulae X-C(=O)-Y-, X-C(=O)-Y-, -Y-C(=O)-O-X, X-C(=S)-Y-, -Y-C(=S)-O-X, -Y-C(=S)-S-X, X-C(=N)-Y-, XZN-, X-S-, X-SO₂-, X-SO₂-NR₁₀-, 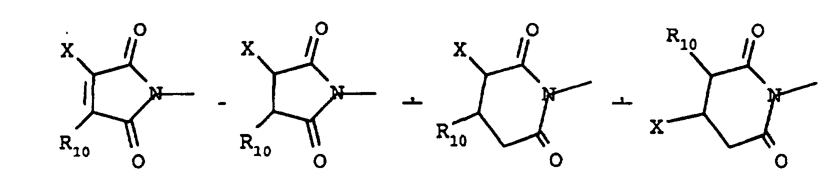 and -N(R₁₀)-C(=O)-O-X; wherein X and Z are each hydrogen or an aliphatic, alicyclic, araliphatic, aromatic or heterocyclic radical, or X and Z together form a 3 to 8 membered ring, Y is the residue of an active methylene containing moiety, and R₁₀ is hydrogen, an aliphatic radical, an alicyclic radical, an araliphatic radical, an aromatic radical or a heterocyclic radical, or R₁₀ and X together form a 3 to 8 membered ring, and the precursor which reacts with a hydroxyl group to form an -O- linkage is selected form the group consisting of an alkyl halide and radicals of the formulae X-C(=O)O-, X-C(=S)O-, X-SO₂-O- and XO-; wherein X has the meaning given above.
which diketopyrrolopyrrole derivative contains from 0 to 6 moles of -SO₃M per mole of diketopyrrolopyrrole derivative; wherein M is hydrogen or a metal or ammonium cation, which process comprises a reaction wherein a 1,4-diketo-3,6-diarylpyrrolopyrrole of the formula 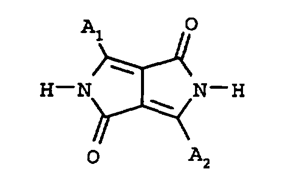 is reacted in a first step with formaldehyde to yield a sulfonated or non-sulfonated intermediate of the formula 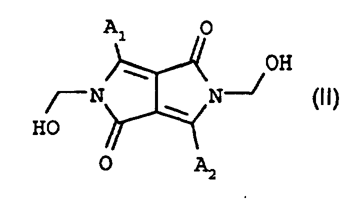 which reacts in a second step with a precursor of the organic radicals, B₁ and B₂, toyield the diketopyrrolopyrrole derivative of formula (I).

2. A process for the preparation of a diketopyrrolopyrrole derivative of the formula (I) wherein A₁ and A₂ are radicals of the formula 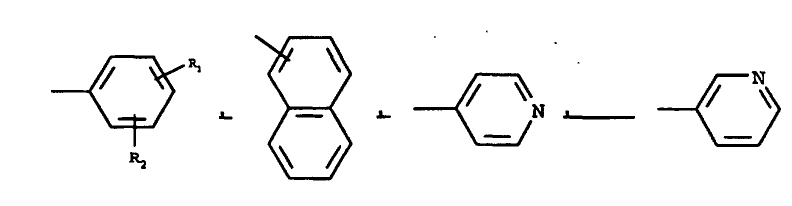 or 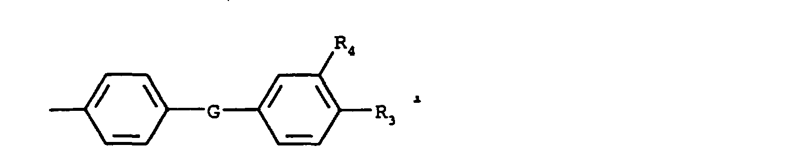 wherein
R₁ and R₂ are each independently of the other hydrogen, chloro, bromo, C₁-C₄alkyl, C₁-C₆alkoxy, C₁-C₆alkylamino, phenyl or CN,
G is -O-, -NR₇-, -N=N- or -SO₂-,
R₃ and R₄ are hydrogen, and
R₇ is hydrogen, methyl or ethyl.

3. A process of claim 1 or 2 wherein the precursor of the organic radicals, B ₁ and B₂, is a 1,4-diketo-3,6-diarylpyrrolo[3,4-c]pyrrole or a quinacridone.

4. A compound of the formula (II) 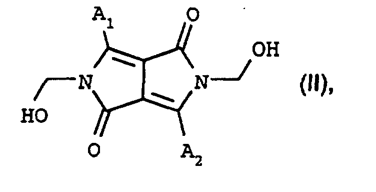 which contains from 0 to 6 moles of -SO₃M per mole of the compound, M is hydrogen or a metal or ammonium cation and A₁ and A₂ are as defined in claim 1.

5. A process for preparing a compound of the formula (II) 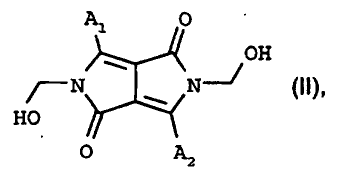 which contains from 0 to 6 moles of -SO₃M per mole of the compound, wherein A₁ and A₂ are as defined in claim 4 and M is hydrogen or a metal or ammonium cation, which process comprises reacting a 1,4-diketo-3,6-diarylpyrrolopyrrole of the formula 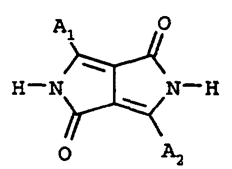 with formaldehyde or paraformaldehyde.

6. A diketopyrrolopyrrole derivative of the formula (la) 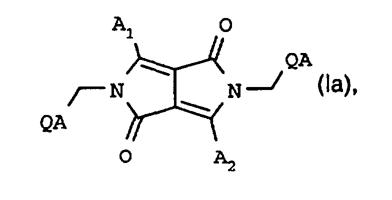 wherein A₁ and A₂ are as defined in claim 1; and each QA is a quinacridone radical; which diketopyrrolopyrrole derivative is substituted by from 0 to 6 moles of -SO₃M per mole of diketopyrrolopyrrole derivative; wherein M is hydrogen or a metal or ammonium cation.

7. A diketopyrrolopyrrole derivative of claim 6 wherein each QA is a quinacridone radical of the formula (III) 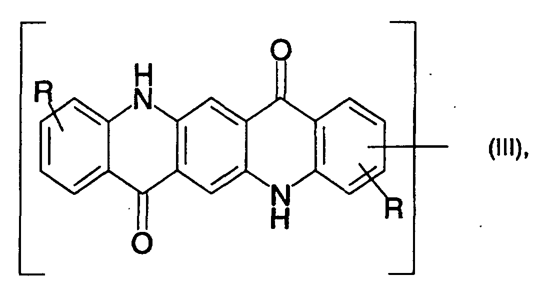 wherein each R is independently hydrogen, halogen, carboxyl, unsubstituted C₁-C₆ alkyl, C₁-C₆ alkyl which is substituted by halogen, unsubstituted C₁-C₆ alkoxy, C₁-C₆ alkoxy which is substituted by halogen.

8. A diketopyrrolopyrrole derivative of the formula (Ib) 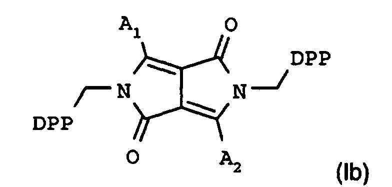 wherein A₁ and A₂ are as defined in claim 1; and each DPP is a diketodiarylpyrrolopyrrole radical; which diketopyrrolopyrrole derivative is substituted by from 0 to 6 moles of -SO₃M per mole of diketopyrrolopyrrole derivative, wherein M is hydrogen or a metal or ammonium cation.

9. A pigment composition which comprises an organic pigment and a diketopyrrolopyrrole derivative of formulae (1), (la) or (lb) according to claims 1, 6, 7 or 8.

10. A method of reducing the viscosity of a dispersion of an organic pigment in a high-molecular-weight organic compound, which comprises incorporating an effective viscosity-reducing amount of a diketopyrrolopyrrole derivative of formulae (I), (la) or (lb) according to claims 1, 6, 7 or 8 into the dispersion.

11. A method of preparing a basecoat/clearcoat finish, which comprises the step of applying a dispersion comprising an organic pigment and an effective viscosity-reducing amount of the diketopyrrolopyrrole derivative of formulae (I), (la) or (lb) according to claims 1, 6, 7 or 8 in a high-molecular-weight organic compound to a substrat.

12. A method of preparing a basecoat/clearcoat finish, which comprises the step of applying a dispersion comprising an effective viscosity-reducing and saturation-enhancing amount of a diketopyrrolopyrrole derivative of formula (Ib) according to claim 8 in a high-molecular-weight organic compound to a substrat.

## Patentansprüche

1. Verfahren zur Herstellung eines Diketopyrrolopyrrol-Derivats der Formel (I) 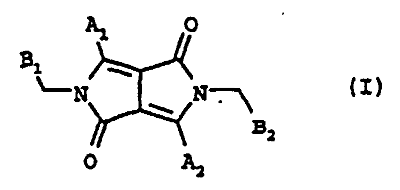 worin A₁ und A₂ Gruppen der Formel 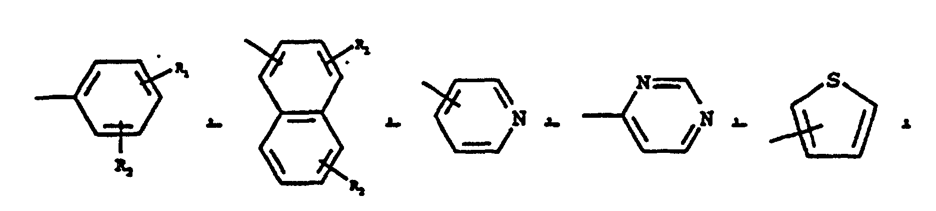 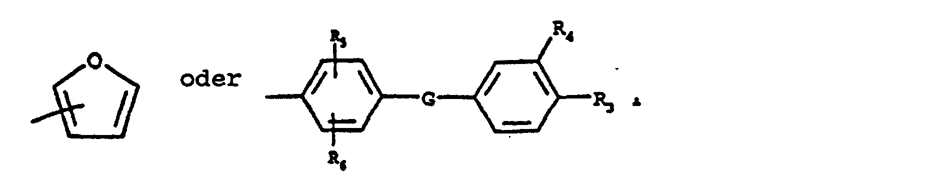 sind, worin
R₁ und R₂ jeweils unabhängig voneinander Wasserstoff, Halogen, C₁-C₁₈-Alkyl, C₁-C₁₈-Alkoxy, C₁-C₁₈-Alkylmercapto, C₁-C₁₈-Alkylamino, C₁-C₁₈-Alkoxycarbonyl, C₁-C₁₈-Alkylaminocarbonyl, -CN, -NO₂, Trifluoromethyl, C₅-C₆-Cycloalkyl, -C=N-(C₁-C₁₈-Alkyl), Phenyl, 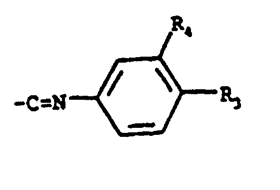 Imidazolyl, Pyrrazolyl, Triazolyl, Piperazinyl, Pyrrolyl, Oxazolyl, Benzoxazolyl, Benzothiazolyl, Benzimidazolyl, Morpholinyl, Piperidinyl oder Pyrrolidinyl sind;
G -CH₂-, -CH(CH₃)-, -C(CH₃)₂-, -CH=N-, -N=N-, -O-, -S-, -SO-, -SO₂-, -CONH- oder -NR₇ ist,
R₃ und R₄ jeweils unabhängig voneinander Wasserstoff, Halogen, C₁-C₆-Alkyl, C₁-C₁₈-Alkoxy oder -CN sind; R₅ und R₆ jeweils unabhängig voneinander Wasserstoff, Halogen oder C₁-C₆-Alkyl sind und R₇ Wasserstoff oder C₁-C₆-Alkyl ist;
und B₁ und B₂ identisch oder unterschiedliche organische Gruppen sind, die von einer Vorstufe stammen, die mit den Hydroxygruppen einer Verbindung der Formel (II) entweder durch Substitutionsreaktion oder unter Bildung einer -O-Bindung reagiert; wobei die Vorstufe, die mit einer Hydroxylgruppe durch eine Substitutionsreaktion reagiert, eine Verbindung ist, die aus der Gruppe bestehend aus einer aromatischen Gruppe, einer heteroaromatischen Gruppe oder Gruppen der Formeln X-C(=O)-Y-, X-C(=O)-Y-, -Y-C(=O)-O-X, X-C(=S)-Y-, -Y-C(=S)-O-X, -Y-C(=S)-S-X, X-C(=N)-Y-, XZN-, X-S-, X-SO₂-, X-SO₂-NR₁₀-, 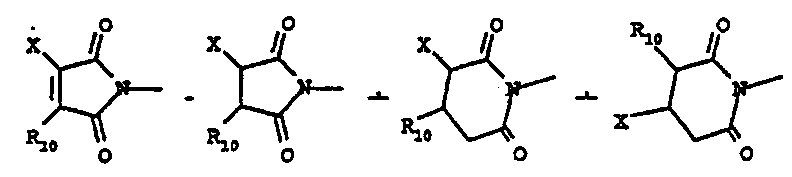 und -N(R₁₀)-C(=O)-O-X ausgewählt wird; worin X und Z jeweils Wasserstoff oder eine aliphatische, alicyclische, araliphatische, aromatische oder heterocyclische Gruppe sind, oder X und Z zusammen einen 3- bis 8-gliedrigen Ring bilden, Y der Rest einer aktives Methylen enthaltenden Gruppierung ist, und R₁₀ Wasserstoff, eine aliphatische Gruppe, eine alicyclische Gruppe, eine araliphatische Gruppe, eine aromatische Gruppe oder eine heterocyclische Gruppe ist oder R₁₀ und X zusammen einen 3- bis 8-gliedrigen Ring bilden; und die Vorstufe, die mit einer Hydroxylgruppe unter Bildung einer -O-Bindung reagiert, aus der Gruppe bestehend aus einem Alkylhalogenid und Gruppierungen der Formeln X-C(=O)O-, X-C(=S)O-, X-SO₂-O- und XO- ausgewählt wird; worin X die oben gegebene Bedeutung hat;
wobei das Diketopyrrolopyrrol-Derivat O bis 6 mol -SO₃M pro mol Diketopyrrolopyrrol-Derivat enthält, worin M Wasserstoff oder ein Metall- oder Ammoniumkation ist;
wobei das Verfahren eine Reaktion umfasst, in der ein 1,4-Diketo-3,6-diarylpyrrolopyrrol der Formel 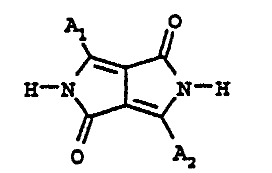 in einer ersten Stufe mit Formaldehyd umgesetzt wird, wobei ein sulfoniertes oder nicht-sulfoniertes Zwischenprodukt der Formel 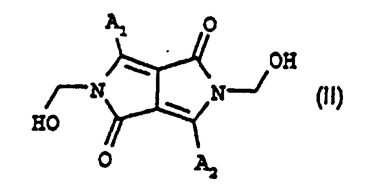 erhalten wird, das in einer zweiten Stufe mit einer Vorstufe der organischen Gruppen B₁ und B₂ unter Erhalt des Diketopyrrolopyrrol-Derivats der Formel (I) reagiert.

2. Verfahren zur Herstellung eines Diketopyrrolopyrrol-Derivats der Formel (I), worin A₁ und A₂ Gruppen der Formel 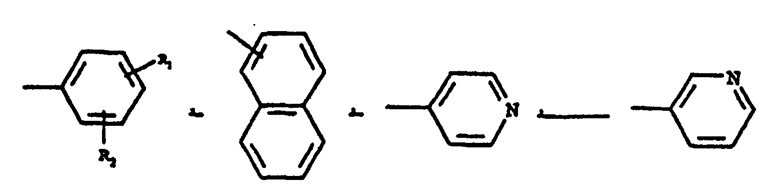 oder 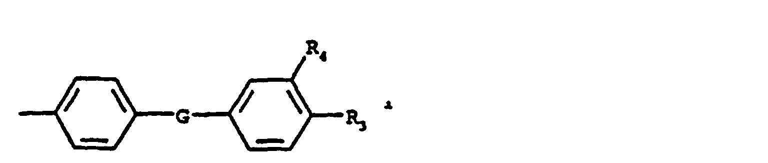 sind, worin
R₁ und R₂ jeweils unabhängig voneinander Wasserstoff, Chlor, Brom, C₁-C₄-Alkyl, C₁-C₆-Alkoxy, C₁-C₆-Alkylamino, Phenyl oder CN sind,
G -O-, -NR₇-, -N=N- oder -SO₂- ist,
R₃ und R₄ Wasserstoff sind und
R₇ Wasserstoff, Methyl oder Ethyl ist.

3. Verfahren nach Anspruch 1 oder Anspruch 2, wobei die Vorstufe der organischen Gruppen, B₁ und B₂, ein 1,4-Diketo-3,6-diarylpyrrolo[3,4-c]pyrrol oder ein Chinacridon ist.

4. Verbindung der Formel (II) 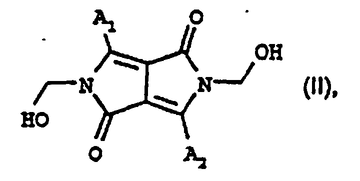 die 0 bis 6 mol -SO₃M pro mol der Verbindung enthält, worin M Wasserstoff oder ein Metall- oder ein Ammoniumkation ist und A₁ und A₂ wie in Anspruch 1 definiert sind.

5. Verfahren zur Herstellung einer Verbindung der Formel (II) 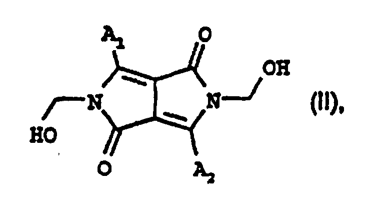 die 0 bis 6 mol -SO₃M pro mol der Verbindung enthält, worin A₁ und A₂ wie in Anspruch 4 definiert sind und M Wasserstoff oder ein Metall- oder Ammoniumkation ist, wobei das Verfahren Umsetzen eines 1,4-Diketo-3,6-diarylpyrrolopyrrol der Formel 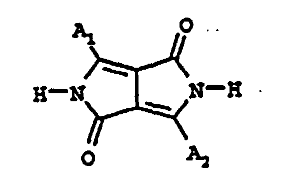 mit Formaldehyd oder Paraformaldehyd umfasst.

6. Diketopyrrolopyrrol-Derivat der Formel (Ia) 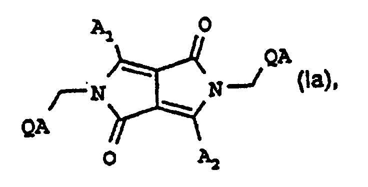 worin A₁ und A₂ wie in Anspruch 1 definiert sind und jedes QA eine Chinacridongruppe ist, wobei das Diketopyrrolopyrrol-Derivat mit 0 bis 6 mol -SO₃M pro mol Diketopyrrolopyrrol-Derivat substituiert ist, worin M Wasserstoff oder ein Metall- oder Ammoniumkation ist.

7. Diketopyrrolopyrrol-Derivat nach Anspruch 6, worin jedes QA eine Chinacridongruppe der Formel (III) ist, 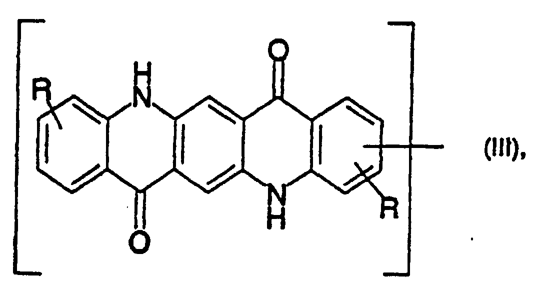 worin jedes R in unabhängiger Weise Wasserstoff, Halogen, Carboxyl, unsubstituiertes C₁-C₆-Alkyl, C₁-C₆-Alkyl, das mit Halogen substituiert ist, unsubstituiertes C₁-C₆-Alkoxy, C₁-C₆-Alkoxy, das mit Halogen substituiert ist, ist.

8. Diketopyrrolopyrrol-Derivat der Formel (Ib) 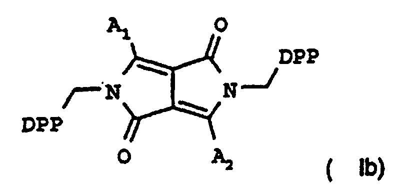 worin A₁ und A₂ wie in Anspruch 1 definiert sind, und jedes DPP eine Diketodiarylpyrrolopyrrol-Gruppe ist, wobei das Diketopyrrolopyrrol-Derivat mit 0 bis 6 mol -SO₃M pro mol Diketopyrrolopyrrol-Derivat substituiert ist, worin M Wasserstoff oder ein Metall- oder Ammoniumkation ist.

9. Pigmentzusammensetzung, die ein organisches Pigment und ein Diketopyrrolopyrrol-Derivat der Formeln (I), (Ia) oder (Ib) nach Anspruch 1, 6, 7 oder 8 umfasst.

10. Verfahren zur Verringerung der Viskosität einer Dispersion eines organischen Pigments in einer organischen Verbindung mit hohem Molekulargewicht, das Einarbeiten einer effektiven die Viskosität verringernden Menge eines Diketopyrrolopyrrol-Derivats der Formeln (I), (Ia) oder (Ib) nach Anspruch 1, 6, 7 oder 8 in die Dispersion umfasst.

11. Verfahren zur Herstellung eines Grundierung/Klarlack-Finishs, das die Stufe eines Auftragens einer Dispersion, die ein organisches Pigment und eine effektive die Viskosität verringernde Menge des Diketopyrrolopyrrol-Derivats der Formeln (I), (Ia) oder (Ib) nach Anspruch 1, 6, 7 oder 8 in einer organischen Verbindung mit hohem Molekulargewicht umfasst, auf ein Substrat umfasst.

12. Verfahren zur Herstellung eines Grundierung/Klarlack-Finishs, das die Stufe eines Auftragens einer Dispersion, die eine effektive die Viskosität verringernde und Sättigung erhöhende Menge eines Diketopyrrolopyrrol-Derivats der Formel (Ib) nach Anspruch 8 in einer organischen Verbindung mit hohem Molekulargewicht umfasst, auf ein Substrat umfasst.

## Revendications

1. Procédé pour la préparation d'un dérivé du dicétopyrrolopyrrole de formule (I) 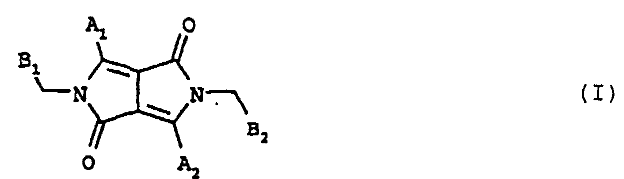 où A₁ et A₂ représentent des radicaux de formule 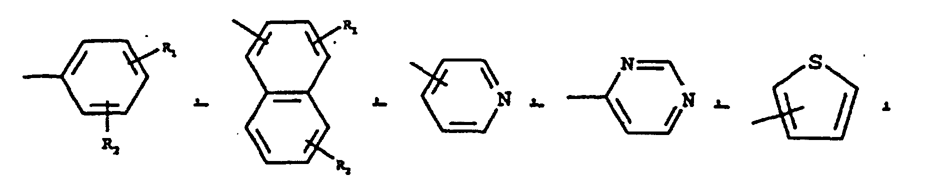 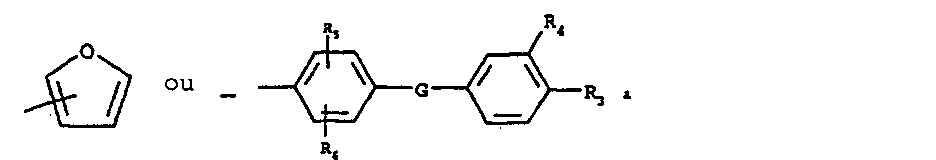 où
R₁ et R₂ représentent chacun, indépendamment l'un de l'autre, un atome d'hydrogène, un atome d'halogène, des groupes alkyle en C₁-C₁₈, alkoxy en C₁-C₁₈, (alkyl en C₁-C₁₈)mercapto, (alkyl en C₁-C₁₈)amino, (alkoxy en C₁-C₁₈)carbonyle, (alkyl en C₁-C₁₈)aminocarbonyle, -CN, -NO₂, trifluorométhyle, cycloalkyle en C₅-C₆, -C=N-(alkyle en C₁-C₁₈), phényle, 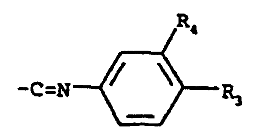 imidazolyle, pyrrazolyle, triazolyle, pipérazinyle, pyrrolyle, oxazolyle, benzoxazolyle, benzothiazolyle, benzimidazolyle, morpholinyle, pipéridinyle ou pyrrolidinyle,
G représente -CH₂-, -CH(CH₃)-, -C(CH₃)₂-, -CH=N-, -N=N-, -O-, -S-, -SO-, -SO₂-, -CONH- ou -NR₇-,
R₃ et R₄ représentent chacun, indépendamment l'un de l'autre, un atome d'hydrogène, un atome d'halogène, des groupes alkyle en C₁-C₆, alkoxy en C₁-C₁₈ ou -CN, R₅ et R₆ représentent chacun, indépendamment l'un de l'autre, un atome d'hydrogène, un atome d'halogène ou un groupe alkyle en C₁-C₆ et R₇ représente un atome d'hydrogène ou un groupe alkyle en C₁-C₆, et B₁ et B₂ sont des radicaux organiques identiques ou différents dérivant d'un précurseur qui réagit sur les groupes hydroxy d'un composé de formule (II) soit par une réaction de substitution, soit pour former une liaison -O- ; où le précurseur qui réagit sur le groupe hydroxyle par une réaction de substitution est un composé pris dans le groupe comportant un radical aromatique, un radical hétéroaromtique ou des radicaux de formules X-C(=O)-Y-, X-C(=O)-Y-, -Y-C(=O)-O-X, X-C(=S)-Y-, -Y-C(=S)-O-X, -Y-C(=S)-S-X, X-C(=N)-Y-, XZN-, X-S-, X-SO₂-, X-SO₂-NR₁₀-, 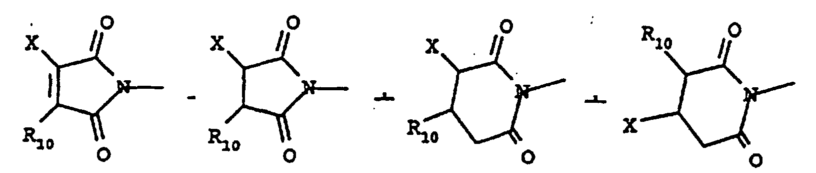 et N(R₁₀)-C(=O)-O-X ; où X et Z représentent chacun un atome d'hydrogène ou un radical aliphatique, alicyclique, araliphatique, aromatique ou hétérocyclique, ou X et Z ensemble forment un cycle de 3 à 8 chaînons, Y représente le résidu d'un fragment présentant un groupe méthylène actif et R₁₀ représente un atome d'hydrogène, un radical aliphatique, un radical alicyclique, un radical araliphatique, un radical aromatique ou un radical hétérocyclique, ou R₁₀ et X forment conjointement un cycle de 3 à 8 chaînons, et le précurseur qui réagit sur groupe hydroxyle pour former une liaison -O- est pris dans le groupe comportant un halogénure d'alkyle et des radicaux de formules X-C(=O)O-, X-C(=S)O-, -X-SO₂-O- et XO- ; où X possède la signification donnée ci-dessus ; ledit dérivé du dicétopyrrolopyrrole contenant de 0 à 6 moles de groupes -SO₃M par mole de dérivé du dicétopyrrolopyrrole ; où M représente un atome d'hydrogène ou un cation métal ou ammonium, ledit procédé comprenant une réaction dans laquelle un 1,4-dicéto-3,6-diarylpyrrolopyrrole de formule 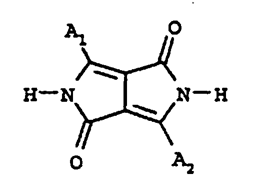 réagit dans une première étape sur le formaldéhyde pour donner un intermédiaire sulfoné ou non sulfoné de formule 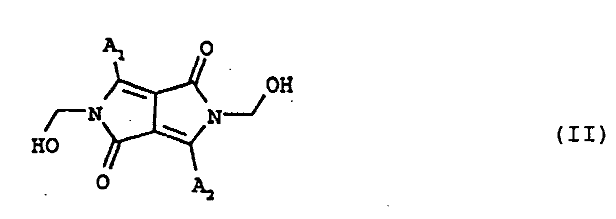 qui réagit dans une seconde étape sur un précurseur des radicaux organiques B₁ et B₂ pour donner le dérivé du dicétopyrrolopyrrole de formule (I).

2. Procédé pour la préparation d'un dérivé du dicétopyrrolopyrrole de formule (I), où A₁ et A₂ représentent des radicaux de formule 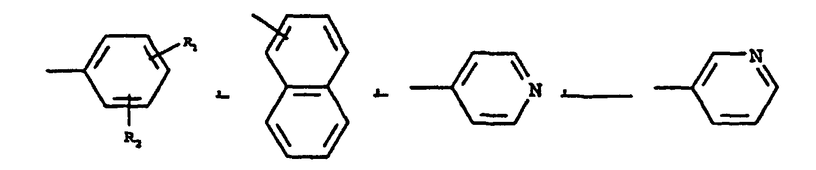 ou 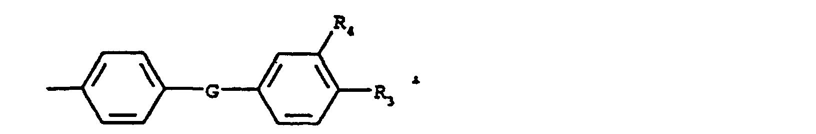 où R₁ et R₂ représentent chacun, indépendamment l'un de l'autre, un atome d'hydrogène, des groupes chloro, bromo, alkyle en C₁-C₄, alkoxy en C₁-C₆, (alkyl en C₁-C₆)amino, phényle ou CN,
G représente des groupes -O-, -NR₇-, -N=N- ou -SO₂-,
R₃ et R₄ représentent des atomes d'hydrogène et
R₇ représente un atome d'hydrogène, des groupes méthyle ou éthyle.

3. Procédé de la revendication 1 ou 2, où le précurseur des radicaux organiques, B₁ et B₂, est un 1,4-dicéto-3,6-diarylpyrrolo[3,4-c]pyrrole ou un quinacridone.

4. Composé de formule (II) 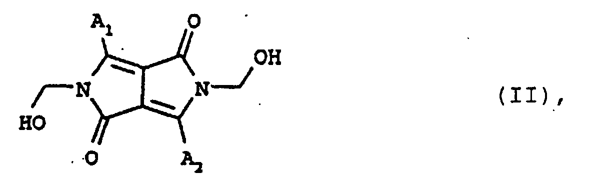 qui présente une teneur de 0 à 6 moles en groupes -SO₃M par mole du composé, M représente un atome d'hydrogène ou un cation métal ou ammonium et A₁ et A₂ sont tels que définis à la revendication 1.

5. Procédé de préparation d'un composé de formule (II) 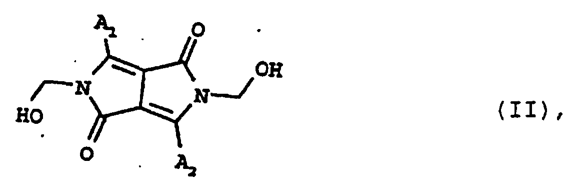 qui présente une teneur de 0 à 6 moles en groupes -SO₃M par mole du composé, où A₁ et A₂ sont tels que définis à la revendication 4 et M représente un atome d'hydrogène ou un cation métal ou ammonium, procédé comprenant la réaction d'un 1,4-dicéto-3,6-diarylpyrrolopyrrole de formule 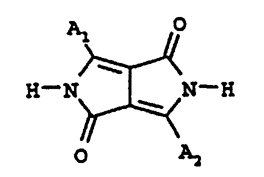 sur le formaldéhyde ou paraformaldéhyde.

6. Dérivé du dicétopyrrolopyrrole de formule (Ia) 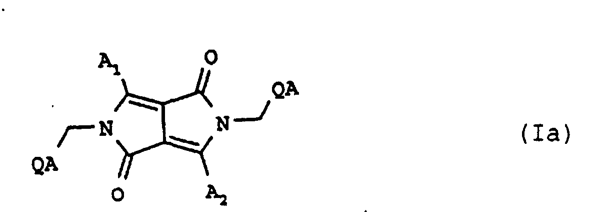 où A₁ et A₂ sont tels que définis comme à la revendication 1 ; et chaque QA représente un radical quinacridone ; lequel dérivé du dicétopyrrolopyrrole est substitué par 0 à 6 moles de groupes -SO₃M par mole du dérivé du dicétopyrrolopyrrole ; où M représente un atome d'hydrogène ou un cation métal ou ammonium.

7. Dérivé du dicétopyrrolopyrrole de la revendication 6, où ,chaque QA est un radical quinacridone de formule (III) 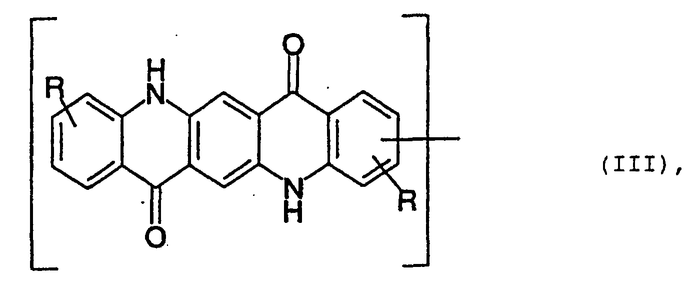 où chaque R représente, indépendamment, un atome d'hydrogène, un atome d'halogène, des groupes carboxyle, alkyle en C₁-C₆ non substitué, alkyle en C₁-C₆ substitué par un substituant halogène, alkoxy en C₁-C₆ non substitué, alkoxy en C₁-C₆ substitué par un substituant halogène.

8. Dérivé du dicétopyrrolopyrrole de formule (Ib) 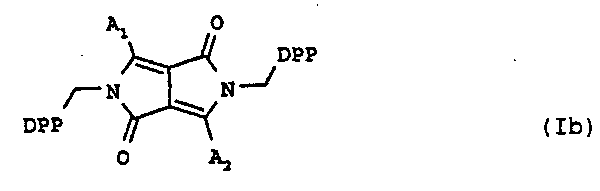 où A₁ et A₂ sont tels que définis comme à la revendication 1; et chaque DPP est un radical dicétodiarylpyrrolopyrrole ; lequel dérivé du dicétopyrrolopyrrole est substitué par 0 à 6 moles de groupes -SO₃M par mole du dérivé du dicétopyrrolopyrrole, où M représente un atome d'hydrogène ou un cation métal ou ammonium.

9. Composition pigmentaire comprenant un pigment organique et un dérivé du dicétopyrrolopyrrole de formules (I), (Ia) ou (Ib) conformément aux revendications 1, 6, 7 ou 8.

10. Procédé pour la réduction de la viscosité d'une dispersion d'un pigment organique dans un composé organique de haut poids moléculaire comprenant l'incorporation d'une quantité efficace pour réduire la viscosité d'un dérivé du dicétopyrrolopyrrole de formules (I), (Ia) ou (Ib) conformément aux revendications 1, 6, 7 ou 8 dans la dispersion.

11. Procédé pour la préparation d'une finition couche de fond/couche claire qui comprend l'étape d'application d'une dispersion comprenant un pigment organique et une quantité du dérivé du dicétopyrrolopyrrole de formules (I), (Ia) ou (Ib) efficace pour réduire la viscosité conformément aux revendications 1, 6, 7 ou 8 dans un composé organique de haut poids moléculaire, à un substrat.

12. Procédé pour la préparation d'une finition couche de fond/couche claire, qui comprend l'étape d'application d'une dispersion comprenant une quantité efficace de réduction de viscosité et d'augmentation de saturation d'un dérivé du dicétopyrrolopyrrole de formule (Ib) conformément à la revendication 8 dans un composé organique de haut poids moléculaire, à un substrat.
